(19) Europäisches Patentamt
European Patent Office
Office européen des brevets

(11) Veröffentlichungsnummer: **0 034 831**
**B1**

# (12) EUROPÄISCHE PATENTSCHRIFT

(45) Veröffentlichungstag der Patentschrift: **12.12.84**

(21) Anmeldenummer: **81101321.8**

(22) Anmeldetag: **24.02.81**

(51) Int. Cl.³: **C 07 D 521/00,** A 61 K 31/395 // C07C143/68 ,(C07D521/00, 231/12, 233/60, 235/06, 249/08, 231/16, 233/56, 249/18, 231/56, 249/04, 231/14)

(54) **Substituierte Phenoxy-aminopropanol-Derivate, Zwischenprodukte und Verfahren zu deren Herstellung sowie diese enthaltende Arzneimittel.**

(30) Priorität: **25.02.80 GB 8006261**
***10.11.80 GB 8035997***

(43) Veröffentlichungstag der Anmeldung: **02.09.81 Patentblatt 81/35**

(45) Bekanntmachung des Hinweises auf die Patenterteilung: **12.12.84 Patentblatt 84/50**

(84) Benannte Vertragsstaaten:
**AT BE CH DE FR GB IT LI LU NL SE**

(56) Entgegenhaltungen:
**DE - A - 2 619 164**
**DE - A - 2 926 517**
**FR - A - 2 455 587**
**FR - M - 2 414**

***Bull. Soc. Chim. Fr., 1973, Seiten 1179-1183***
***Eur. I. Med. Chem. - Chimica Therapeutica, 1979, S. 231-237***

(73) Patentinhaber: **F. HOFFMANN-LA ROCHE & CO. *Aktiengesellschaft, CH-4002 Basel (CH)***

(72) Erfinder: **Machin, Peter James, 8 The Elms Tooting Bec Road, London SW17 8BT (GB)**

(74) Vertreter: **Lederer, Franz, Dr. et al, Patentanwälte Dr. Franz Lederer Reiner F. Meyer Lucile-Grahn-Strasse 22, D-8000 München 80 (DE)**

**EP 0 034 831 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft substituierte Phenoxy-aminopropanol-Derivate. Im speziellen betrifft sie substituierte Phenoxy-aminopropanol-Derivate, ein Verfahren für ihre Herstellung und diese enthaltende pharmazeutische Präparate.

Die erfindungsgemäßen substituierten Phenoxy-aminopropanol-Derivate sind Verbindungen der allgemeinen Formel

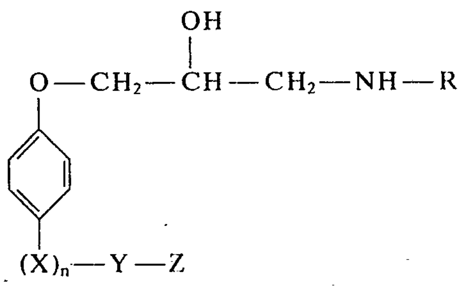

(I)

worin R niederes Alkyl, X ein Sauerstoff- oder Schwefelatom, n die Zahl 0 oder 1, Y Methylen, Äthylen, Propylen oder, wenn n die Zahl 0 bedeutet, auch die Gruppe

$$-CH{=}CH-\overset{*}{C}H_2- \qquad (a)$$

wobei die Doppelbindung die trans-Konfiguration aufweist und das mit einem Stern bezeichnete Kohlenstoffatom mit der Gruppe Z verknüpft ist, und Z einen von einem Stickstoffatom aus an die Gruppe Y gebundenen 5-gliedrigen, aromatischen, heterocyclischen Ring, der eines oder mehrere Stickstoffatome als einzige(s) Heteroatom(e) enthält und der durch Halogen, niederes Alkyl, niederes Alkoxy, Phenyl, Cyano oder Carboxamido substituiert sein kann, oder an zwei benachbarten Kohlenstoffatomen mit einem Rest der Formel

$$-(CH_2)_4- \qquad (b)$$

oder

$$-CH{=}CH-CH{=}CH- \qquad (c)$$

verknüpft sein kann, bedeuten,
und pharmazeutisch annehmbare Säureadditionssalze davon.

Der in dieser Beschreibung verwendete Ausdruck »niederes Alkyl« bezeichnet geradkettige oder verzweigte Alkylgruppen, welche 1—4 Kohlenstoffatome enthalten (z. B. Methyl, Äthyl, Propyl, Isopropyl, Butyl und t-Butyl). Der Ausdruck »niederes Alkoxy« bedeutet geradkettige oder verzweigte Alkoxygruppen, welche 1—4 Kohlenstoffatome enthalten (z. B. Methoxy, Äthoxy, Propoxy, Isopropoxy, etc.). Der Ausdruck »Halogen« bedeutet Fluor, Chlor, Brom und Jod. Beispiele für die mit Z bezeichneten aromatischen, heterocyclischen Ringe sind 1-Imidazolyl, 1 H-1,2,4-Triazol-1-yl, 1 H-1,2,3-Triazol-1-yl, 2 H-1,2,3-Triazol-2-yl, 1-Pyrazolyl, 4-Halo-1-pyrazolyl (z. B. 4-Chor-1-pyrazolyl), 4-Phenyl-1-pyrazolyl, 1-Benzimidazolyl, 2 H-Benzotriazol-2-yl, 4,5,6,7-Tetrahydro-2 H-benzotriazol-2-yl, 1 H-Indazol-1-yl und dergleichen.

Eine bevorzugte Klasse von Verbindungen der allgemeinen Formel I umfaßt solche, worin R Isopropyl oder t-Butyl bedeutet, wobei Isopropyl bevorzugt ist, X ein Sauerstoffatom, n die Zahl 1 und Y Methylen bedeuten. Ebenfalls bevorzugt sind Verbindungen der allgemeinen Formel I, worin Z 1-Imidazolyl 1 H-1,2,4-Triazol-1-yl, 2 H-1,2,3-Triazol-2-yl, 1-Pyrazolyl oder 4-Halo-1-pyrazolyl bedeutet, wobei Z besonders bevorzugt 2 H-1,2,3-Triazol-2-yl oder 4-Chlor-1-pyrazolyl bedeutet. Aus dem Obigen folgt, daß eine ganz besonders bevorzugte Klasse von Verbindungen der allgemeinen Formel I, solche umfaßt, worin R Isopropyl, X ein Sauerstoffatom, n die Zahl 1, Y Methylen und Z 2 H-1,2,3-Triazol-2-yl oder 4-Chlor-1-pyrazolyl bedeuten.

Besonders bevorzugte Verbindungen der allgemeinen Formel I sind:

1-Isopropylamino-3-[4-[2-(1-pyrazolyl)äthoxy]phenoxy]-2-propanol,
1-[4-[2-(1-Imidazolyl)äthoxy]phenoxy]-3-isopropylamino-2-propanol,
1-[4-[2-(1-Benzimidazolyl)äthoxy]phenoxy]-3-isopropylamino-2-propanol,
1-Isopropylamino-3-[4-[2-(1 H-1,2,4-triazol-1-yl)äthoxy]phenoxy]-propanol,
1-t-Butylamino-3-[4-[2-(1-pyrazolyl)äthoxy]phenoxy]-2-propanol,
1-Isopropylamino-3-[4-[3-(1-pyrazolyl)propoxy]phenoxy]-2-propanol,

1-Isopropylamino-3-[4-[3-(1 H-1,3,4-triazol-1-yl)-propoxy]-phenoxy]-2-propanol,
1-Isopropylamino-3-[4-[2-(4-phenyl-1-pyrazolyl)äthoxy]phenoxy]-2-propanol,
1-Isopropylamino-3-[4-[2-(4-chlor-1-pyrazolyl)äthoxy]phenoxy]-2-propanol,
1-[4-[2-(1-Imidazolyl)äthyl]phenoxy]-3-isopropylamino-2-propanol,
1-Isopropylamino-3-[4-[2-(1-pyrazolyl)äthyl]phenoxy]-2-propanol,
1-Isopropylamino-3-[4-[2-(1 H-1,2,4-triazol-1-yl)äthyl]phenoxy]-2-propanol,
1-Isopropylamino-3-[4-[(1-pyrazolyl)methyl]phenoxy]-2-propanol,
1-[4-[2-(1-Imidazolyl)methyl]phenoxy]-3-isopropylamino-2-propanol,
1-[4-[2-(2 H-Benzotriazol-2-yl)äthoxy]phenoxy]-3-isopropylamino-2-propanol,
1-[4-[2-(4,5,6,7-Tetrahydro-2 H-benzotriazol-2-yl)äthoxy]phenoxy]-2-propanol,
1-Isopropylamino-3-[4-[3-(1-pyrazolyl)propyl]phenoxy]-2-propanol,
trans-1-Isopropylamino-3-[4-[3-(1-pyrazolyl)-1-propyl]phenoxy]-2-propanol,
1-Isopropylamino-3-[4-[2-(1-pyrazolyl)äthylthio]phenoxy]-2-propanol und
1-[4-[2-(1 H-Indazol-1-yl)äthoxy]phenoxy]-3-isopropylamino-2-propanol.

Weitere besonders bevorzugte Verbindungen der allgemeinen Formel I sind:

1-Isopropylamino-3-[4-[2-(2 H-1,2,3-triazol-2-yl)äthoxy]phenoxy]-2-propanol,
1-Isopropylamino-3-[4-[(1-pyrazolyl)methoxy]phenoxy]-2-propanol,
1-[4-[2-Hydroxy-3-(isopropylamino)propoxy]phenoxymethyl]-4-pyrazol-carbonitril,
1-Isopropylamino-3-[4-[(1 H-1,2,3-triazol-1-yl)methoxy]phenoxy]-2-propanol und
1-Isopropylamino-3-[4-[(1-pyrazolyl)methylthio]phenoxy]-2-propanol.

Ganz besonders bevorzugte Verbindungen der allgemeinen Formel I sind:

1-[4-[(4-Chlor-1-pyrazolyl)methoxy]phenoxy]-3-isopropylamino-2-propanol und
1-Isopropylamino-3-[4-[(2 H-1,2,3-triazol-2-yl)methoxy]phenoxy]-2-propanol.

Die substituierten Phenoxy-aminopropanol-Derivate der allgemeinen Formel I und ihre pharmazeutisch annehmbaren Säureadditionssalze können erfindungsgemäß dadurch hergestellt werden, daß man

a) ein Epoxid der allgemeinen Formel

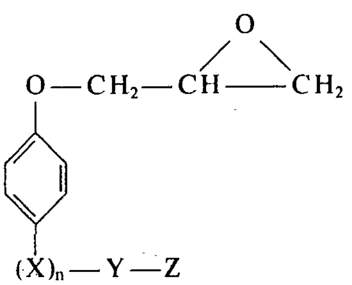

(II)

worin X, Y, Z und n obige Bedeutung besitzen,
mit einem Amin der allgemeinen Formel

$R—NH_2$ (III)

worin R obige Bedeutung besitzt,
umsetzt, oder

b) zur Herstellung einer Verbindung der allgemeinen Formel I, worin X ein Sauerstoffatom, n die Zahl 1 und Y Methylen, Äthylen oder Propylen bedeuten, ein Alkalimetall-Derivat eines Phenols der allgemeinen Formel

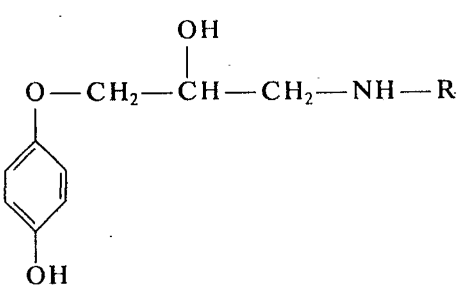

(IV)

worin R obige Bedeutung besitzt,
mit einer Verbindung der allgemeinen Formel

$$R^1—Y^1—Z \qquad (V)$$

worin $R^1$ eine Abgangsgruppe und $Y^1$ Methylen, Äthylen oder Propylen bedeuten, und Z obige Bedeutung besitzt,
umsetzt, und

c) erwünschtenfalls ein erhaltenes Racemat in die optischen Antipoden spaltet, und/oder
d) erwünschtenfalls eine erhaltene Verbindung der allgemeinen Formel I in ein pharmazeutisch annehmbares Säureadditionssalz überführt.

Die Behandlung eines Epoxides der Formel II mit einem Amin der Formel III (das eine bekannte Verbindung ist oder nach an sich bekannten Methoden hergestellt werden kann), gemäß Verfahrensvariante a), kann in an sich bekannter Weise durchgeführt werden. Die Reaktion kann in Gegenwart oder in Abwesenheit eines inerten organischen Lösungsmittels durchgeführt werden. Verwendet man ein inertes organisches Lösungsmittel, so kommen beispielsweise niedere Alkohole, wie Methanol, Äthanol oder dergleichen, in Frage. Andererseits kann auch überschüssiges Amin der Formel III als Lösungsmittel dienen. Vorteilhafterweise arbeitet man in einem Temperaturbereich von etwa 0°C bis etwa Raumtemperatur, vorzugsweise bei Raumtemperatur, und bei Atmosphärendruck.

Die mit $R^1$ bezeichnete Abgangsgruppe in einer Verbindung der Formel V kann irgendeine herkömmliche Abgangsgruppe sein; beispielsweise ein Chlor- oder Bromatom, eine niedere Alkylsulfonyloxygruppe (z. B. Methansulfonyloxy) oder eine Arylsulfonyloxygruppe (z. B. p-Toluolsulfonyloxy). $R^1$ bedeutet vorzugsweise eine Arylsulfonyloxygruppe, insbesondere p-Toluolsulfonyloxy.

Die Reaktion eines Alkalimetall-Derivates eines Phenols der Formel IV mit einer Verbindung der Formel V, gemäß Verfahrensvariante b), wird zweckmäßigerweise in einem inerten organischen Lösungsmittel durchgeführt. Inerte organische Lösungsmittel, welche verwendet werden können, sind beispielsweise Dimethylformamid, Dioxan, Dimethoxyäthan und Tetrahydrofuran, wobei Dimethylformamid bevorzugt wird. Das Alkalimetall-Derivat eines Phenols der Formel IV wird vorzugsweise in situ aus einem entsprechenden Phenol und einem Alkalimetall, einem Alkalimetallhydrid oder einem Alkalimetallamid, vorzugsweise einem Alkalimetallhydrid, insbesondere Natriumhydrid, hergestellt. Die Reaktion wird vorteilhafterweise bei erhöhter Temperatur, vorzugsweise bei etwa 60°C, durchgeführt.

Es sei besonders hervorgehoben, daß die Verbindungen der allgemeinen Formel I ein asymmetrisch substituiertes Kohlenstoffatom besitzen, und somit in racemischen oder optisch aktiven Formen auftreten können. Die vorliegende Erfindung umfaßt sowohl die Racemate als auch die optisch aktiven Formen. Gemäß Verfahrensvariante c) kann ein erhaltenes Racemat erwünschtenfalls nach an sich bekannten Methoden in die optischen Antipoden gespalten werden; beispielsweise durch fraktionierte Kristallisation von Salzen, welche mit optisch aktiven Säuren erhalten wurden. Es sei noch erwähnt, daß man das als Ausgangsmaterial verwendete Phenol der Formel IV auch in optisch aktiver Form einsetzen kann, und somit eine optisch aktive Verbindung der Formel I erhält.

Gemäß Verfahrensvariante d) können die Verbindungen der Formel I durch Behandeln mit einer pharmazeutisch annehmbaren anorganischen Säure (z. B. Salzsäure, Bromwasserstoffsäure, Schwefelsäure, Phosphorsäure, etc.) oder mit einer pharmazeutisch annehmbaren organischen Säure (z. B. Essigsäure, Weinsäure, Zitronensäure, Fumarsäure, Maleinsäure, Apfelsäure, Methansulfonsäure, p-Toluolsulfonsäure, etc.) in pharmazeutisch annehmbare Säureadditionssalze übergeführt werden.

Die in Verfahrensvariante a) als Ausgangsstoffe verwendeten Epoxide der Formel II sind neu und ebenfalls Gegenstand der vorliegenden Erfindung. Sie können hergestellt werden, indem man ein Alkalimetall-Derivat eines Phenols der allgemeinen Formel

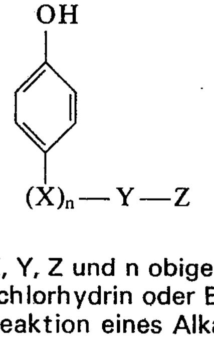

(VI)

worin X, Y, Z und n obige Bedeutung besitzen,
mit Epichlorhydrin oder Epibromhydrin umsetzt.

Die Reaktion eines Alkalimetall-Derivates eines Phenols der Formel VI mit Epichlorhydrin oder Epibromhydrin, vorzugsweise mit Epichlorhydrin, kann in Analogie zur Reaktion eines Alkalimetall-Derivates eines Phenols der Formel IV mit einer Verbindung der Formel V durchgeführt werden.

Die Phenole der Formel VI, worin X, Y, Z und n die obige Bedeutung haben, mit der Maßgabe, daß Z

nicht 1-Imidazolyl bedeutet, wenn n die Zahl 0 und Y Methylen, Äthylen oder Propylen bedeuten, und daß Z nicht 1-Benzimidazolyl bedeutet, wenn n die Zahl 0 und Y Methylen bedeuten (4-(1-Imidazolylmethyl)phenol und 4-(1-Benzimidazolylmethyl)phenol sind bekannt, vgl. Bull. Soc. Chim. Fr., 1973, Seiten 1179–1183, und Eur. J. Med. Chem. – Chimica Therapentica, 1979, Seiten 231–237), sind neu und ebenfalls Gegenstand der vorliegenden Erfindung. Sie können je nach der Bedeutung von X, Y und n auf verschiedenen Wegen hergestellt werden.

Phenole der Formel VI, worin X ein Sauerstoffatom, Y Äthylen oder Propylen und n die Zahl 1 bedeuten, können beispielsweise gemäß folgendem Reaktionsschema I, worin Z obige Bedeutung besitzt, $Y^2$ Äthylen oder Propylen und Bz Benzyl bedeuten, hergestellt werden.

Reaktionsschema I

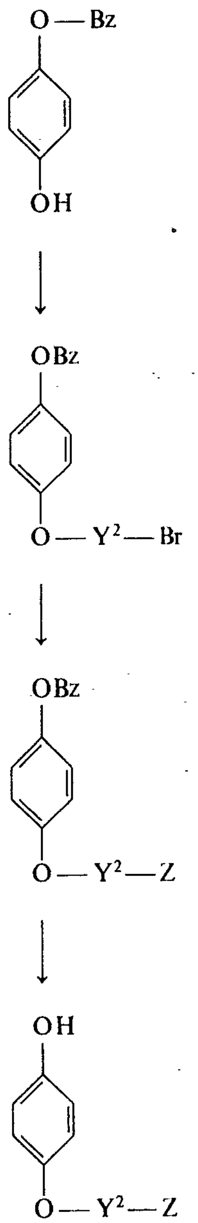

(VII)

(VIII)

(IX)

(VIa)

Gemäß Reaktionsschema I wird 4-Benzyloxyphenol der Formel VII mit einer Verbindung der allgemeinen Formel

$$Br—Y^2—Br \qquad (X)$$

worin $Y^2$ obige Bedeutung besitzt, umgesetzt, wobei man eine Verbindung der Formel VIII erhält. Diese Reaktion wird in an sich bekannter Weise durchgeführt; beispielsweise in einem wäßrigen alkalischen Medium, wie wäßriges

Alkalimetallhyroxid (z. B. wäßrige Natronlauge) bei erhöhter Temperatur (z. B. bei etwa 100°C) durchgeführt.

Eine Verbindung der Formel VIII wird anschließend in eine Verbindung der Formel IX übergeführt, und zwar durch Reaktion mit einer heterocyclischen Verbindung der allgemeinen Formel

$$H—Z \qquad (XI)$$

worin Z obige Bedeutung besitzt.

Diese Reaktion wird in an sich bekannter Weise durchgeführt; beispielsweise in Gegenwart eines inerten organischen Lösungsmittels, wie Dimethylformamid und dergleichen, und in Gegenwart einer geeigneten Alkalimetallbase, wie Alkalimetallhydrid (z. B. Natriumhydrid). Die Reaktion wird zweckmäßigerweise bei erhöhter Temperatur (z. B. bei etwa 60°C) durchgeführt.

Schließlich wird eine Verbindung der Formel IX durch Debenzylierung in das gewünschte Phenol der Formel VIa übergeführt. Die Debenzylierung kann in an sich bekannter Weise durchgeführt werden; beispielsweise unter Verwendung von Wasserstoff in Gegenwart eines Katalysators (z. B. Palladium/Kohle und dergleichen) oder unter Verwendung von Bromwasserstoff in Eisessig.

Phenole der Formel VI, worin X ein Sauerstoffatom und n die Zahl 1 bedeuten, können beispielsweise auch hergestellt werden, indem man ein Alkalimetall-Derivat von 4-Benzyloxyphenol der Formel VII mit einer Verbindung der Formel V, vorzugsweise einem Chlorid, umsetzt und das erhaltene Produkt in Analogie zur Debenzylierung einer Verbindung der Formel IX debenzyliert.

Das folgende Reaktionsschema II erläutert die Herstellung von Phenolen der Formel VI, worin n die Zahl 0 und Y Äthylen bedeuten. In diesem Reaktionsschema besitzen Z und Bz obige Bedeutung, und $R^2$ bedeutet niederes Alkyl oder Aryl.

Reaktionsschema II

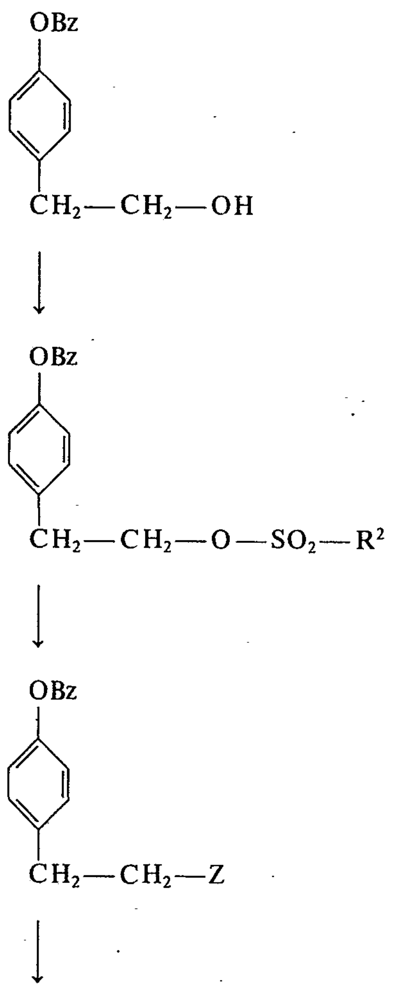

(XII)

(XIII)

(XIV)

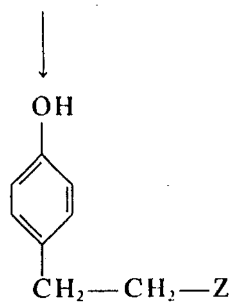

(VIb)

Gemäß Reaktionsschema II wird 4-Benzyloxyphenäthylalkohol der Formel XII durch Umsetzen mit einem niederen Alkylsulfonyl- oder Arylsulfonylhalogenid, vorzugsweise Methansulfonylchlorid, in eine Verbindung der Formel XIII übergeführt. Die Reaktion wird zweckmäßigerweise in Gegenwart eines inerten organischen Lösungsmittels (z. B. eines Äthers, wie Diäthyläther) und in Gegenwart eines säurebindenden Mittels, vorzugsweise einer tertiären organischen Base, wie Pyridin usw., durchgeführt. Vorteilhafterweise wird die Reaktion bei einer Temperatur unterhalb Raumtemperatur, insbesondere bei etwa 0°C, durchgeführt.

Eine erhaltene Verbindung der Formel XIII wird anschließend durch Umsetzen mit einer heterocyclischen Verbindung der Formel XI in Analogie zur weiter oben beschriebenen Reaktion einer Verbindung der Formel VIII mit einer solchen heterocyclischen Verbindung in eine Verbindung der Formel XIV übergeführt.

Durch Debenzylierung einer Verbindung der Formel XIV, in Analogie zur weiter oben beschriebenen Debenzylierung einer Verbindung der Formel IX, erhält man das gewünschte Phenol der Formel VI b.

Phenole der Formel VI, worin n die Zahl 0 und Y Propylen bedeuten, können beispielsweise wie in folgendem Reaktionsschema III, worin Z und Bz obige Bedeutung besitzen, erläutert hergestellt werden.

Reaktionsschema III

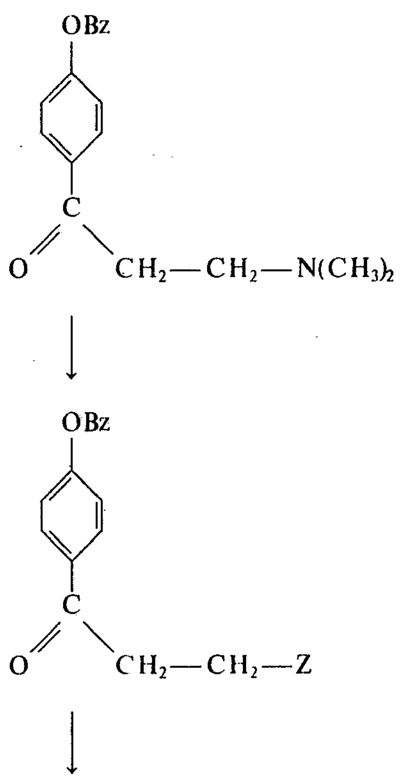

(XV)

(XVI)

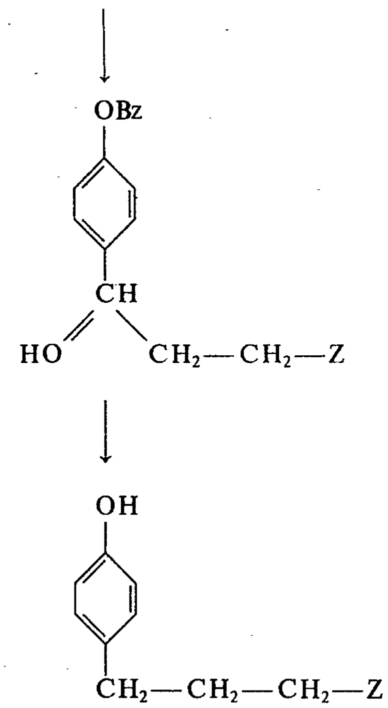

(XVII)

(VIc)

Gemäß Reaktionsschema III wird in einem ersten Schritt 1-Dimethylamino-3,4-benzyloxyphenyl-propan-3-on der Formel XV mit einer heterocyclischen Verbindung der Formel XI zu einer Verbindung der Formel XVI umgesetzt. Diese Reaktion wird in herkömmlicher Weise durchgeführt; beispielsweise durch gemeinsames Erhitzen der Reaktionspartner in einem geeigneten Alkohol (z. B. N-Butanol). Die Reaktion wird vorteilhafterweise bei der Rückflußtemperatur der Reaktionsmischung durchgeführt.

Eine erhaltene Verbindung der Formel XVI wird anschließend durch Reduktion in an sich bekannter Weise in eine Verbindung der Formel XVII übergeführt. Die Reduktion kann beispielsweise mit einem Alkalimetallborhydrid, wie Natriumborhydrid, in Gegenwart eines inerten organischen Lösungsmittels, wie einem niederen Alkohol (z. B. Äthanol), bei etwa Raumtemperatur durchgeführt werden.

Schließlich erhält man das gewünschte Phenol der Formel VI c durch katalytische Hydrierung einer Verbindung der Formel XVII unter sauren Bedingungen. Die katalytische Hydrierung kann in herkömmlicher Weise in einem inerten organischen Lösungsmittel (z. B. in einem niederen Alkohol, wie Methanol), beispielsweise unter Verwendung von Palladium/Kohle oder dergleichen als Katalysator, durchgeführt werden. Die sauren Bedingungen können beispielsweise durch Salzsäure hergestellt werden.

Phenole der Formel VI, worin n die Zahl 0 und Y Methylen bedeuten, können beispielsweise hergestellt werden, indem man 4-Hydroxybenzylalkohol in an sich bekannter Weise mit einer heterocyclischen Verbindung der Formel XI erhitzt (z. B. auf etwa 160°C).

Phenole der Formel VI, worin X ein Schwefelatom und n die Zahl 1 bedeuten, können beispielsweise hergestellt werden, indem man 4-Mercaptophenol mit einer Verbindung der Formel V umsetzt.

Die Reaktion von 4-Mercaptophenol mit einer Verbindung der Formel V kann in herkömmlicher Weise durchgeführt werden; beispielsweise in Gegenwart eines inerten organischen Lösungsmittels, wie Dimethylformamid und dergleichen, und in Gegenwart einer geeigneten Alkalimetallbase, wie eines Alkalimetallhydrides (z. B. Natriumhydrid) oder eines Alkalimetallcarbonates (z. B. Kaliumcarbonat) bei etwa Raumtemperatur.

Phenole der Formel VI, worin n die Zahl 0 und Y einen Rest der Formel (a) bedeuten, können beispielsweise wie im nachfolgenden Reaktionsschema IV, worin Z und Bz obige Bedeutung besitzen und Et Äthyl bedeutet, erläutert hergestellt werden.

Reaktionsschema IV

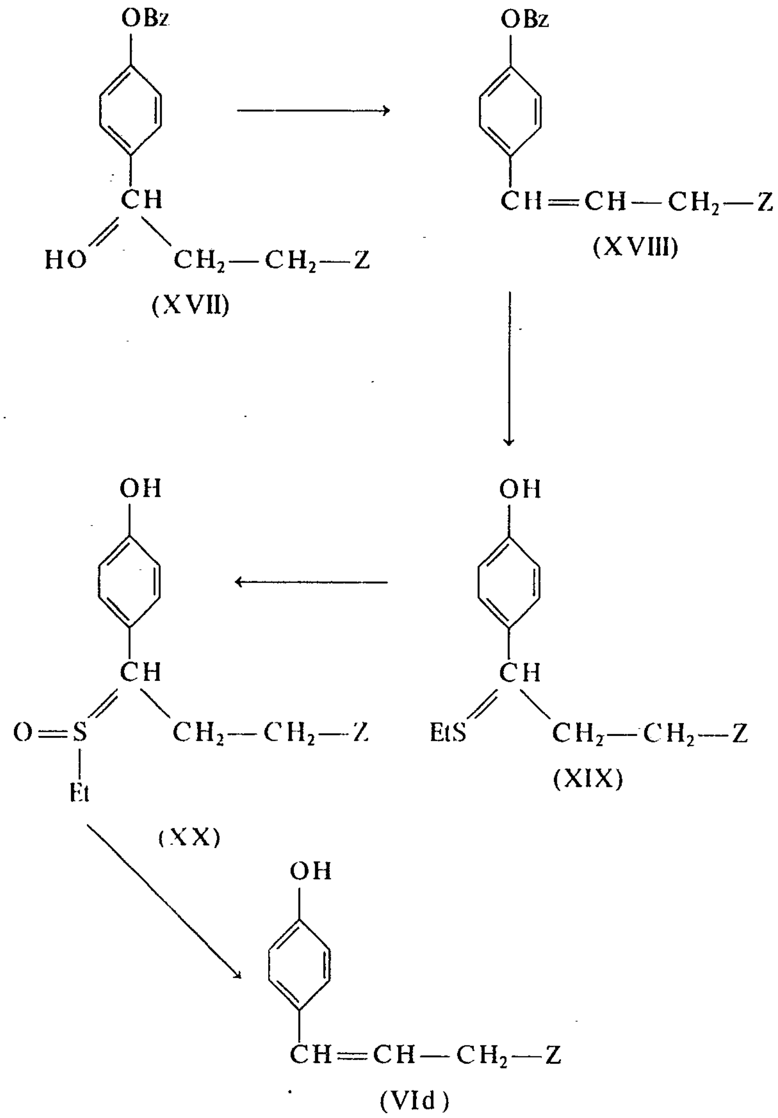

(XVII) (XVIII) (XIX) (XX) (VId)

Gemäß Reaktionsschema IV wird eine Verbindung der Formel XVII, hergestellt wie weiter oben in Reaktionsschema III beschrieben, zuerst in eine Verbindung der Formel XVIII übergeführt, und zwar durch Dehydratisierung, welche beispielsweise so durchgeführt werden kann, daß man eine Verbindung der Formel XVII mit einem niederen Alkylsulfonyl- oder Arylsulfonylchlorid, vorzugsweise Methansulfonylchlorid, in Gegenwart einer tertiären organischen Base, vorzugsweise Pyridin, erhitzt. Die Reaktion wird vorteilhafterweise bei etwa 100°C durchgeführt.

Eine erhaltene Verbindung der Formel XVIII wird anschließend durch Behandeln mit Äthanthiol und Bortrifluorid-diäthylätherat in eine Verbindung der Formel XIV übergeführt. Die Reaktion wird vorteilhafterweise bei etwa Raumtemperatur durchgeführt.

Die Oxidation einer Verbindung der Formel XIX zu einer Verbindung der Formel XX wird vorteilhafterweise mit einer organischen Persäure in Gegenwart eines inerten organischen Lösungsmittels durchgeführt. Geeignete organische Persäuren, welche verwendet werden können, sind Peressigsäure, Perbenzosäure, halogenierte Perbenzosäuren, vorzugsweise m-Chlorperbenzoesäure, Monoperphthalsäure, etc. Als inerte organische Lösungsmittel kommen beispielsweise halogenierte Kohlenwasserstoffe, wie Methylenchlorid oder dergleichen, in Frage.

Schließlich wird eine Verbindung der Formel XX durch Erhitzen in einem inerten organischen Lösungsmittel in das gewünschte Phenol der Formel VI d übergeführt. Die bevorzugten inerten organischen Lösungsmittel sind aromatische Kohlenwasserstoffe, insbesondere Toluol. Die Reaktion wird zweckmäßigerweise bei der Rückflußtemperatur der Reaktionsmischung durchgeführt.

Die in den weiter oben beschriebenen Verfahren zur Herstellung von Phenolen der Formel VI verwendeten Ausgangsstoffe sind bekannte Verbindungen, oder können in Analogie zu den bekannten Verbindungen in an sich bekannter Weise hergestellt werden.

Das in Verfahrensvariante b) als Ausgangsmaterial verwendete Phenol der Formel IV sowie die Verbindungen der Formel V sind bekannte Verbindungen oder können in Analogie zu den bekannten Verbindungen in an sich bekannter Weise hergestellt werden.

Die erfindungsgemäßen substituierten Phenoxy-aminopropanol-Derivate besitzen cardioselektive β-adrenergische Blockereigenschaften und können demnach bei der Bekämpfung bzw. Verhütung von Herzkrankheiten, wie beispielsweise Angina pectoris und Herzrhythmusstörungen, verwendet werden. Sie können auch als antihypertensive Wirkstoffe verwendet werden.

Die β-adrenergischen Blockereigenschaften der vorliegenden, substituierten Phenoxy-aminopropanol-Derivate an $\beta_1$- und $\beta_2$-Adrenalin-Rezeptoren können mittels Standardtestverfahren nachgewiesen werden. In einem solchen Testverfahren werden diese Eigenschaften an Ratten dadurch gezeigt, daß man diejenige Dosierung der zu testenden Verbindung in μ/kg i. v. ermittelt, welche notwendig ist, um die durch Isoprenalin induzierte Tachycardie [$ED_{50}$ (HR)] und die durch Isoprenalin induzierte Blutdrucksenkungsreaktion [$ED_{50}$ (BD)] um 50% zu reduzieren. Ist für eine getestete Verbindung die $ED_{50}$ (BD) signifikant höher als die $ED_{50}$ (HR), so blockiert diese Verbindung selektiver die $\beta_1$-Adrenalin-Rezeptoren als die $\beta_2$-Adrenalin-Rezeptoren (d. h. sie ist cardioselektiv). Die im vorliegenden Test mit repräsentativen Vertretern der erfindungsgemäßen, substituierten Phenoxy-aminopropanol-Derivate und dem Atenolol (ein allgemein bekanntes und häufig verwendetes cardioselektives β-adrenergisches Blockermittel) erhaltenen Resultate sind in der folgenden Tabelle zusammengefaßt.

Tabelle

| Derivat | $ED_{50}$ (HR) (μg/kg i.v.) | $ED_{50}$ (BD) (μg/kg i.v.) |
|---|---|---|
| A | 50 | 6500 |
| B | 4 | 2430 |
| C | 11 | 2000 |
| D | 14 | 2000 |
| Atenolol | 91 | 2130 |

Derivat A: 1-Isopropylamino-3-[4-[2-(1-pyrazolyl)-äthoxy]-phenoxy]-2-propanol-Hydrogen-maleat.
Derivat B: 1-[4-[(4-Chlor-1-pyrazolyl)methoxy]-phenoxy]-3-isopropylamino-2-propanol-Hydrogen-oxalat.
Derivat C: 1-Isopropylamino-3-[4-[(2H-1,2,3-triazol-2-yl)methoxy]phenoxy]-2-propanol-Hydrochlorid.
Derivat D: 1-Isopropylamino-3-[4-[2-(2H-1,2,3-triazol-2-yl)äthoxy]phenoxy]-2-propanol-Hydrochlorid.

Die erfindungsgemäßen substituierten Phenoxy-aminopropanol-Derivate können als Arzneimittel in Form von pharmazeutischen Präparaten, welche diese in Kombination mit einem verträglichen pharmazeutischen Trägermaterial enthalten, verwendet werden. Dieses Trägermaterial kann ein inertes organisches oder anorganisches Trägermaterial sein, das für die enterale (z. B. orale) oder parenterale Verabreichung geeignet ist. Als Beispiele solcher Trägermaterialien seien Wasser, Gelatine, Lactose, Stärke, Magnesiumstearat, Talk, Gummi arabicum, Polyalkylenglykole, pflanzliche Öle, Vaselin und dergleichen genannt. Die pharmazeutischen Präparate können in herkömmlicher Weise hergestellt werden und in festen Dosierungsformen (z. B.) als Tabletten, Dragées, Suppositorien oder Kapseln) oder in flüssigen Dosierungsformen (z. B. als Lösungen, Suspensionen oder Emulsionen) vorliegen. Die pharmazeutischen Präparate können herkömmlichen pharmazeutischen Nachbehandlungsmaßnahmen, wie Sterilisation, unterworfen werden. Weiterhin können die pharmazeutischen Präparate herkömmliche Hilfsmittel, wie Konservierungsmittel, Stabilisierungsmittel, Emulgiermittel, Aromastoffe, Netzmittel, Salze, um den osmotischen Druck zu verändern, Puffer, und dergleichen, enthalten.

Ein erfindungsgemäßes, substituiertes Phenoxy-aminopropanol-Derivat kann Erwachsenen in einer täglichen Dosierung von etwa 1 mg pro kg bis 10 mg pro kg in einer einzigen Dosis oder in mehreren

Dosen verabreicht werden. Es sei festgehalten, daß die obigen Dosierungsangaben lediglich als Beispiele dienen, und daß die Dosierungen nach oben oder nach unten, in Abhängigkeit von Faktoren, wie das zu verabreichende, substituierte Phenoxy-aminopropanol-Derivat, die Art der Verabreichung und die Bedürfnisse des Patienten, wie sie vom behandelnden Arzt festgestellt werden, variieren können.

Die folgenden Beispiele erläutern das erfindungsgemäße Verfahren.

Beispiel 1

Eine Lösung von 3,16 g (15 mMol) 4-[2-(1-Pyrazolyl)-äthoxy]phenol in 75 ml Dimethylformamid wird während 5 Minuten zusammen mit 0,72 g (15 mMol) Natriumhydrid (50%ige Öldispersion) gerührt. Man versetzt mit 10 ml Epichlorhydrin und rührt die erhaltene Lösung während 30 Minuten bei 60°. Nach Entfernen des Lösungsmittels und des überschüssigen Epichlorhydrins unter vermindertem Druck wird der Rückstand zwischen Essigester und Wasser verteilt. Die organische Phase wird abgetrennt, über Natriumsulfat getrocknet, filtriert und eingedampft, wobei man 4,1 g kristallines Epoxid erhält, das ohne weitere Reinigung in 100 ml Äthanol, das 15 ml Isopropylamin enthält, aufgelöst wird. Die Mischung wird über Nacht bei Raumtemperatur stehengelassen. Nach Eindampfen der Lösung zur Trockne wird das kristalline Produkt in 100 ml Äthanol, das 1,74 kg (15 mMol) Maleinsäure enthält, aufgelöst. Nach Eindampfen der Lösung wird der Rückstand aus Isopropanol umkristallisiert. Man erhält 5,0 g (76,5 %) 1-Isopropylamino-3-[4-[2-(1-pyrazolyl)äthoxy]phenoxy]-2-propanol-Hydrogenmaleat vom Schmelzpunkt 103—105°.

Das als Ausgangsmaterial verwendete 4-[2-(1-Pyrazolyl)-äthoxy]phenol kann wie folgt hergestellt werden:

a) 125 ml einer 1,6 N-Natronlauge werden über einen Zeitraum von 1 Stunde zu einer heftig gerührten Mischung aus 40 g (0,2 Mol) 4-Benzyloxyphenol und 71,1 g (0,38 Mol) Äthylendibromid bei 100° gegeben. Man rührt noch über Nacht bei 100° und verteilt dann die abgekühlte Mischung zwischen 20%iger wäßriger Natronlauge und Diäthyläther. Die organische Phase wird abgetrennt, mit Wasser gewaschen, über Natriumsulfat getrocknet, filtriert und zur Trockene eingedampft. Durch Umkristallisieren des Rückstandes erhält man 36 g (59 %) 1-Benzyloxy-4-(2-bromäthoxy)-benzol vom Schmelzpunkt 77—80°.

b) Eine Lösung von 1,36 g (20 mMol) Pyrazol in 50 ml Dimethylformamid wird mit 0,96 g (20 mMol) Natriumhydrid (50%ige Öldispersion) versetzt und während 5 Minuten gerührt. Nach Zugabe von 6,14 g (20 mMol) 1-Benzyloxy-4-(2-bromäthoxy)benzol wird die Mischung während 0,5 Stunden unter Rühren auf 60° erwärmt. Nach Entfernen des Lösungsmittels unter vermindertem Druck wird der Rückstand zwischen Essigester und Wasser verteilt. Die organische Phase wird abgetrennt, mit Wasser gewaschen, über Natriumsulfat getrocknet und eingedampft. Man erhält 5,7 g eines kristallinen Rückstandes. Dieses Material wird in 250 ml Äthanol aufgelöst und über Nacht bei Raumtemperatur in Gegenwart von 0,2 g 10%igem Palladium/Kohle hydriert. Nach Abfiltrieren des Katalysators und Eindampfen wird der Rückstand aus Toluol umkristallisiert. Man erhält 3,16 g (77,5 %) 4-[2-(1-Pyrazolyl)äthoxy]phenol vom Schmelzpunkt 74—77°.

Beispiel 2

In Analogie zu den Angaben im ersten Absatz von Beispiel 1 erhält man aus 4-[2-(1-Imidazolyl)-äthoxy]phenol 1-[4-[2-(1-Imidazolyl)äthoxy]phenoxy]-3-isopropylamino-2-propanol-bis-Hydrogenoxalat vom Schmelzpunkt 147—148° (Zersetzung) (aus Methanol).

Das Ausgangsmaterial kann wie folgt hergestellt werden:

In Analogie zu den Angaben in Beispiel 1 b) erhält man aus 1-Benzyloxy-4-(2-bromäthoxy)benzol und Imidazol 4-[2-(1-Imidazolyl)äthoxy]phenol vom Schmelzpunkt 141—144°.

Beispiel 3

In Analogie zu den Angaben im ersten Absatz von Beispiel 1 erhält man aus 4-[2-(1-Benzimidazolyl)äthoxy]phenol 1-[4-[2-(1-Benzimidazolyl)äthoxy]phenoxy]-3-isopropylamino-2-propanol-bis-Hydrogenoxalat vom Schmelzpunkt 170—173° (aus Äthanol).

Das Ausgangsmaterial kann wie folgt hergestellt werden:

In Analogie zu den Angaben in Beispiel 1 b) erhält man aus 1-Benzyloxy-4-(2-bromäthoxy)benzol und Benzimidazol 4-[2-(1-Benzimidazolyl)äthoxy]phenol.

Beispiel 4

In Analogie zu den Angaben im ersten Absatz von Beispiel 1 erhält man aus 4-[2-(1 H-1,2,4-Triazol-1-yl)-äthoxy]phenol 1-Isopropylamino-3-[4-[2-(1 H-1,2,4-triazol-1-yl)äthoxy]phenoxy]-2-propanol-dihydrochlorid vom Schmelzpunkt 153—158° (aus Isopropanol/Äthanol).

Das Ausgangsmaterial kann wie folgt hergestellt werden:

In Analogie zu den Angaben in Beispiel 1 b) erhält man aus 1-Benzyloxy-4-(2-bromäthoxy)benzol und 1 H-1,2,4-Triazol 4-[2-(1 H-1,2,4-triazol-1-yl)äthoxy]phenol.

Beispiel 5

In Analogie zu den Angaben im ersten Absatz von Beispiel 1, jedoch unter Verwendung von t-Butylamin anstelle von Isopropylamin, erhält man 1-t-Butylamino-3-[4-[2-(1-pyrazolyl)äthoxy]phenoxy]-2-propanol-Hydrogenmaleat vom Schmelzpunkt 145—148° (aus Äthanol).

Beispiel 6

In Analogie zu den Angaben im ersten Absatz von Beispiel 1 erhält man aus 4-[3-(1-Pyrazolyl)propoxy]phenol 1-Isopropylamino-3-[4-[3-(1-pyrazolyl)propoxy]phenoxy]-2-propanol-Hydrogenmaleat vom Schmelzpunkt 89—91° (aus Isopropanol).

Das Ausgangsmaterial kann wie folgt hergestellt werden:

a) In Analogie zu den Angaben in Beispiel 1 a) erhält man aus 4-Benzyloxyphenol und 1,3-Dibrompropan 1-Benzyloxy-4-(3-brompropoxy)benzol vom Schmelzpunkt 49—53° (aus Methanol).

b) In Analogie zu den Angaben in Beispiel 1 b) erhält man aus 1-Benzyloxy-4-(3-brompropoxy)benzol und Pyrazol 4-[3-(1-Pyrazolyl)propoxy]phenol vom Schmelzpunkt 108—110°.

Beispiel 7

In Analogie zu den Angaben im ersten Absatz von Beispiel 1 erhält man aus 4-[3-(1 H-1,2,4-Triazol-1-yl)-propoxy]phenol 1-Isopropylamino-3-[4-[3-(1 H-1,2,4-Triazol-1-yl)propoxy]phenoxy]-2-propanol-dihydrochlorid vom Schmelzpunkt 158—160° (aus Äthanol).

Das Ausgangsmaterial kann wie folgt hergestellt werden:

In Analogie zu den Angaben in Beispiel 1 b) erhält man aus 1-Benzyloxy-4-(3-brompropoxy)benzol [hergestellt wie in Beispiel 6 a) beschrieben] und 1 H-1,2,4-Triazol 4-[3-(1 H-1,2,4-Triazol-1-yl)propoxy]phenol vom Schmelzpunkt 141—144°.

Beispiel 8

In Analogie zu den Angaben im ersten Absatz von Beispiel 1 erhält man aus 4-[2-(4-Phenyl-1-pyrazolyl)äthoxy]phenol 1-Isopropylamino-3-[4-[2-(4-phenyl-1-pyrazolyl)äthoxy]phenoxy]-2-propanol-Hydrogenmaleat vom Schmelzpunkt 141—143° (aus Isopropanol).

Das Ausgangsmaterial kann wie folgt hergestellt werden:

Eine Mischung aus 5,7 g (15,4 mMol) 1-Benzyloxy-4-[2-(4-phenyl-1-pyrazolyl)äthoxy]benzol [hergestellt in Analogie zu den Angaben in Beispiel 1 b) aus 1-Benzyloxy-4-(2-bromäthoxy)benzol und 4-Phenylpyrazol] und 20 ml 48%iger Bromwasserstoffsäure in Eisessig wird während 0,5 Stunden bei 25° gerührt. Die erhaltene Lösung wird zur Trockne eingedampft. Der Rückstand wird zwischen 2 N wäßriger Natronlauge und Diäthyläther verteilt. Die wäßrige Phase wird mit konzentrierter Salzsäure auf pH 6 angesäuert und mit Essigester ausgeschüttelt. Die Essigesterauszüge werden gewaschen, über Natriumsulfat getrocknet, filtriert und eingedampft. Der Rückstand wird aus Toluol umkristallisiert und liefert 3,24 g (75%) 4-[2-(4-Phenyl-1-pyrazolyl)äthoxy]phenol vom Schmelzpunkt 146—150°.

Beispiel 9

In Analogie zu den Angaben im ersten Absatz von Beispiel 1 erhält man aus 4-[2-(4-Chlor-1-pyrazolyl)äthoxy]phenol 1-Isopropylamino-3-[4-[2-(4-chlor-1-pyrazolyl)äthoxy]phenoxy]-2-propanol-p-toluolsulfonat vom Schmelzpunkt 120° (aus Äthanol).

Das Ausgangsmaterial kann wie folgt hergestellt werden:

In Analogie zu den Angaben im zweiten Absatz von Beispiel 8 erhält man aus 1-Benzyloxy-4-[2-(4-chlor-1-pyrazolyl)äthoxy]benzol [hergestellt in Analogie zu den Angaben in Beispiel 1 b) aus 1-Benzyl-

oxy-4-(2-bromäthoxy)benzol und 4-Chlorpyrazol] 4-[2-(4-Chlor-1-pyrazolyl)äthoxy]phenol vom Schmelzpunkt 105° (aus Tetrachlorkohlenstoff).

Beispiel 10

In Analogie zu den Angaben im ersten Absatz von Beispiel 1 erhält man aus 2,82 g (15 mMol) 4-[2-(1-Imidazolyl)äthyl]phenol 5,57 g (69 %) 1-[4-[2-(1-Imidazolyl)äthyl]phenoxy]-3-isopropylamino-2-propanol-bis-Hydrogenmaleat vom Schmelzpunkt 104—106° (aus Isopropanol).

Das Ausgangsmaterial kann wie folgt hergestellt werden:

a) Eine Lösung von 3,51 g (30,7 mMol) Methansulfonylchlorid in 5 ml Diäthyläther wird über einen Zeitraum von 0,5 Stunden zu einer gerührten Lösung von 7,0 g (30,7 mMol) 4-Benzyloxyphenäthylalkohol in 20 ml Pyridin bei 0° zugetropft. Man rührt noch während 2 Stunden, wobei man die Temperatur langsam auf Raumtemperatur steigen läßt. Die Mischung wird anschließend zwischen 150 ml 2 N-Salzsäure und Methylenchlorid verteilt. Die organische Phase wird abgetrennt, mit Wasser gewaschen, über Natriumsulfat getrocknet, filtriert und eingedampft. Der Rückstand kristallisiert nach Behandeln mit n-Hexan. Nach Abfiltrieren und Trocknen der Kristalle erhält man 8,9 g (95 %) 1-Benzyloxy-4-(2-methansulfonyloxyäthyl)benzol vom Schmelzpunkt 60—64°.

b) 1,93 g (28 mMol) Imidazol in 100 ml Dimethylformamid werden mit 1,36 g (28 mMol) Natriumhydrid (50%ige Öldispersion) behandelt. Die Mischung wird während 5 Minuten gerührt, mit 8,65 g (28 mMol) 1-Benzyloxy-4-(2-methansulfonyloxyäthyl)benzol versetzt und unter Rühren während 0,5 Stunden auf 60° erhitzt. Man entfernt das Lösungsmittel unter vermindertem Druck und verreibt den Rückstand mit Wasser. Das erhaltene Material wird abfiltriert, mit Wasser gewaschen, getrocknet in 200 ml Äthanol aufgelöst (7,5 g) und über Nacht bei Atmosphärendruck und Raumtemperatur in Gegenwart von 0,2 g 10%igem Palladium/Kohle hydriert. Nach Abfiltrieren des Katalysators und Entfernen des Lösungsmittels wird der Rückstand aus Äthanol umkristallisiert und liefert 3,36 g (67 %) 4-[2-(1-Imidazolyl)äthyl]phenol vom Schmelzpunkt 158—161°.

Beispiel 11

In Analogie zu den Angaben im ersten Absatz von Beispiel 1 erhält man aus 4-[2-(1-Pyrazolyl)-äthyl]phenol 1-Isopropylamino-3-[4-[2-(1-pyrazolyl)äthyl]phenoxy]-2-propanol-p-toluolsulfonat vom Schmelzpunkt 134—135° (aus Isopropanol).

Das Ausgangsmaterial kann wie folgt hergestellt werden:

In Analogie zu den Angaben in Beispiel 10 b) erhält man aus Pyrazol und 1-Benzyloxy-4-(2-methansulfonyloxyäthyl)benzol 4-[2-(1-Pyrazolyl)äthyl]phenol vom Schmelzpunkt 94—95°.

Beispiel 12

In Analogie zu den Angaben im ersten Absatz von Beispiel 1 erhält man aus 4-[2-(1 H-1,2,4-Triazol-yl)äthyl]phenol 1-Isopropylamino-3-[4-[2-(1 H-1,2,4-Triazol-1-yl)äthyl]phenoxy]-2-propanol-dihydrochlorid vom Schmelzpunkt 173—175° (aus Äthanol).

Das Ausgangsmaterial kann wie folgt hergestellt werden:

In Analogie zu den Angaben in Beispiel 10 b) erhält man aus 1 H-1,2,4-Triazol und 1-Benzyloxy-4-(2-methansulfonyloxyäthyl)benzol 4-[2-(1 H-1,2,4-Triazol-1-yl)äthyl]phenol.

Beispiel 13

In Analogie zu den Angaben im ersten Absatz von Beispiel 1 erhält man aus 3,3 g 4-[(1-Pyrazolyl)methyl]phenol 4,0 g (54 %) 1-Isopropylamino-3-[4-[(1-pyrazolyl)methyl]phenoxy]-2-propanol-Hydrogenmaleat vom Schmelzpunkt 103—105° (aus Isopropanol).

Das Ausgangsmaterial kann wie folgt hergestellt werden:

4,96 g (40 mMol) 4-Hydroxy-benzylalkohol und 2,72 g (40 mMol) Pyrazol werden zusammen während 30 Minuten auf 160° erhitzt. Der erhaltene Festkörper wird abgekühlt und in 150 ml siedendem Toluol aufgelöst. Nach abdekantieren von wenig schwarzem Teer wird die Lösung zur Trockne eingedampft, und der Rückstand aus Essigester umkristallisiert. Man erhält 3,3 g (47 %) 4-[(1-Pyrazolyl)methyl]phenol vom Schmelzpunkt 113—115°.

Beispiel 14

In Analogie zu den Angaben im ersten Absatz von Beispiel 1 erhält man aus 4-[(1-Imidazolyl)methyl]-phenol 1-[4-[(1-Imidazolyl)methyl]phenoxy]-3-isopropylamino-2-propanol-bis-Hydrogenoxalat vom Schmelzpunkt 105—107° (aus Methanol).

Das Ausgangsmaterial kann wie folgt hergestellt werden:

In Analogie zu den Angaben im zweiten Absatz von Beispiel 13 erhält man aus 4-Hydroxy-benzylalkohol und Imidazol 4-[(1-Imidazolyl)methyl]phenol.

Beispiel 15

In Analogie zu den Angaben im ersten Absatz von Beispiel 1 erhält man aus 1,26 g 4-[2-(2H-Benzotriazol-2-yl)äthoxy]phenol 1,23 g (62%) 1-[4-[2-(2H-Benzotriazol-2-yl)äthoxy]phenoxy]-3-isopropylamino-2-propanol-hydrochlorid vom Schmelzpunkt 175—176° (aus Methanol).

Das Ausgangsmaterial kann wie folgt hergestellt werden:

a) Eine Lösung von 5,95 g (50 mMol) Benzotriazol in 100 ml Dimethylformamid wird mit 2,40 g (50 mMol) Natriumhydrid (50%ige Öldispersion) versetzt und während 5 Minuten gerührt. Anschließend gibt man 15,35 g (50 mMol) 1-Benzyloxy-4-(2-bromäthoxy)benzol [hergestellt wie in Beispiel 1 a) beschrieben] hinzu und rührt die erhaltene Mischung während 20 Minuten bei 60°. Nach Entfernen des Lösungsmittels unter vermindertem Druck wird der Rückstand zwischen Wasser und Essigester verteilt. Die organische Phase wird abgetrennt, mit Wasser gewaschen, über Natriumsulfat getrocknet und eingedampft. Das erhaltene kristalline Material (17,5 g) wird unter Eluieren mit 10%igem Essigester/Hexan an einer Kieselgelsäule chromatographiert. Nach Eindampfen des Eluates und Umkristallisieren des Rückstandes aus Äthanol erhält man 6,18 g 1-Benzyloxy-4-[2-(2H-Benzotriazol-2-yl)äthoxy]benzol vom Schmelzpunkt 105—107°.
b) 3,1 g (8,9 mMol) 1-Benzyloxy-4-[2-(2H-benzotriazol-2-yl)äthoxy]benzol werden in Analogie zu den Angaben im zweiten Absatz von Beispiel 8 mit 48%igem Bromwasserstoff/Essigsäure debenzyliert. Man erhält 1,26 g (55%) 4-[2-(2H-Benzotriazol-2-yl)äthoxy]phenol vom Schmelzpunkt 98° (aus Isopropanol).

Beispiel 16

In Analogie zu den Angaben im ersten Absatz von Beispiel 1 erhält man aus 2,8 g 4-[2-(4,5,6,7-Tetrahydro-2H-benzotriazol-2-yl)äthoxy]phenol 3,15 g 1-[4-[2-(4,5,6,7-Tetrahydro-2H-benzotriazol-2-yl)äthoxy]phenoxy]-3-isopropylamino-2-propanol-hydrochlorid vom Schmelzpunkt 157—158° (aus Acetonitril).

Das Ausgangsmaterial kann wie folgt hergestellt werden:

5,5 g (16 mMol) 1-Benzyloxy-4-[2-(2H-benzotriazol-2-yl)äthoxy]benzol [hergestellt wie in Beispiel 15 a) beschrieben] werden in Analogie zu den Angaben in Beispiel 1 b) hydriert bis kein Wasserstoff mehr aufgenommen wird. Man erhält 2,8 g (69%) 4-[2-(4,5,6,7-Tetrahydro-2H-benzotriazol-2-yl)äthoxy]phenol vom Schmelzpunkt 93° (aus Toluol).

Beispiel 17

In Analogie zu den Angaben im ersten Absatz von Beispiel 1 erhält man aus 8,5 g 4-[3-(1-Pyrazolyl)-propyl]phenol 10,90 g (60%) 1-Isopropylamino-3-[4-[3-(1-pyrazolyl)propyl]phenoxy]-2-propanol-Hydrogenmaleat vom Schmelzpunkt 126—130° (aus Isopropanol).

Das Ausgangsmaterial kann wie folgt hergestellt werden:

a) Eine Mischung aus 20 g (71 mMol) 1-Dimethylamino-3-(4-benzyloxyphenyl)propan-3-on, 6,8 g (100 mMol) Pyrazol und 250 ml n-Butanol wird während 15 Stunden unter Rückfluß zum Sieden erhitzt und anschließend zur Trockne eingedampft. Der Rückstand wird zwischen Essigester und Wasser verteilt. Die organische Phase wird abgetrennt, über Natriumsulfat getrocknet, filtriert und eingedampft. Nach Umkristallisieren aus Essigester erhält man 15,1 g (70%) 1-(1-Pyrazolyl)-3-(4-benzyloxyphenyl)propan-3-on vom Schmelzpunkt 111—114°.
b) Eine Lösung des obigen Ketons in 500 ml Äthanol wird zusammen mit 2,0 g Natriumborhydrid während 16 Stunden bei Raumtemperatur gerührt. Nach Entfernen des Lösungsmittels wird der Rückstand zwischen Methylenchlorid und Wasser verteilt. Die organische Phase wird über Natriumsulfat getrocknet, abfiltriert und eingedampft. Man erhält 14,9 g (98%) 1-Benzyloxy-4-[1-hydroxy-3-(1-pyrazolyl)propyl]benzol vom Schmelzpunkt 80—85°.
c) Eine Lösung des obigen Alkohols in 600 ml Äthanol, das 8 ml konzentrierte Salzsäure enthält, wird bei Atmosphärendruck und Raumtemperatur in Gegenwart von 0,4 g 10%igem Palladium/Kohle

hydriert. Nach Abfiltrieren des Katalysators und Eindampfen wird der Rückstand zwischen Essigester und gesättigter Natriumbicarbonatlösung verteilt. Die organische Phase wird abgetrennt, über Natriumsulfat getrocknet, filtriert und eingedampft. Man erhält 8,5 g (87 %) 4-[3-(1-Pyrazolyl)propyl]phenol als farbloses Öl, das ohne weitere Reinigung verwendet wird.

Beispiel 18

113 mg (0,5 mMol) 1-Isopropylamino-3-(4-hydroxyphenoxy)-2-propanol in 1 ml Dimethylformamid werden mit 24 mg (0,5 mMol) Natriumhydrid (50%ige Öldispersion) behandelt. Die Mischung wird während 5 Minuten gerührt, mit 133 mg (0,5 mMol) 1-[2-(p-Toluolsulfonyloxy)äthyl]pyrazol versetzt und unter Rühren während 20 Minuten auf 60° erhitzt. Die Mischung wird zur Trockne eingedampft und der Rückstand zwischen 2 N-Natronlauge und Methylenchlorid verteilt. Die organische Phase wird abgetrennt, mit Wasser gut gewaschen, über Natriumsulfat getrocknet, filtriert und eingedampft. Man erhält kristallines 1-Isopropylamino-3-[4-[2-(1-pyrazolyl)äthoxy]phenoxy]-2-propanol das in 154 mg (71 %) des entsprechenden Maleates übergeführt wird. Dieses Salz ist mit dem in Absatz 1 des ersten Beispiels erhaltenen Materials identisch.

Das als Ausgangsmaterial verwendete 1-[2-(p-Toluolsulfonyloxy)äthyl]pyrazol kann wie folgt hergestellt werden:

a) 34 g (0,5 Mol) Pyrazol und 50 g (0,57 Mol) Äthylencarbonat werden zusammen während 1 Stunde auf 160° erhitzt. Das Produkt, 1-(2-Hydroxyäthyl)pyrazol, wird im Hochvakuum direkt aus der Reaktionsmischung destilliert: Siedepunkt 80—85°/0,5 mm Hg. Ausbeute: 52 g (93 %).

b) 50 g (0,45 Mol) 1-(2-Hydroxyäthyl)pyrazol in 500 ml Pyridin werden über einen Zeitraum von 20 Minuten portionsweise mit insgesamt 85,05 g (0,45 Mol) p-Toluolsulfonylchlorid behandelt. Die Mischung wird während 2 Stunden bei 25° gerührt und anschließend eingedampft. Der Rückstand wird zwischen konzentrierter Salzsäure und Diäthyläther verteilt. Die wäßrige Phase wird mit gesättigter Natriumbicarbonatlösung vorsichtig basisch gestellt und anschließend mit Essigester extrahiert. Die Essigesterauszüge werden mit 1 N-Salzsäure und anschließend mit Wasser gewaschen, über Natriumsulfat getrocknet, filtriert und eingedampft. Man erhält einen tiefschmelzenden festen Rückstand. Nach Verreiben des Rückstandes mit Diäthyläther bei −30° werden die erhaltenen Kristalle abfiltriert. Man erhält 46 g (39 %) 1-[2-(p-Toluolsulfonyloxy)-äthyl]pyrazol vom Schmelzpunkt 37—39°.

Beispiel 19

In Analogie zu den Angaben im ersten Absatz von Beispiel 1 erhält man aus 0,55 g [4-[3-(1-Pyrazolyl)prop-1-enyl]phenol 0,76 g (64 %) trans-1-Isopropylamino-3-[4-[3-(1-pyrazolyl)-1-propenyl]-phenoxy]-2-propanol-Hydrogenmaleat vom Schmelzpunkt 121—125° (aus Isopropanol).

Das Ausgangsmaterial kann wie folgt hergestellt werden:

a) 8,8 g (28,5 mMol) 1-Benzyloxy-4-[1-hydroxy-3-(1-pyrazolyl)propyl]benzol und 3,27 g Methansulfonylchlorid werden während 15 Stunden in Pyridin unter Rückfluß zum Sieden erhitzt. Anschließend versetzt man mit einem weiteren Äquivalent (3,27 g) Methansulfonylchlorid und erhitzt noch einmal 15 Stunden unter Rückfluß zum Sieden. Nach Entfernen des Pyridins wird der Rückstand zwischen Essigester und Wasser verteilt. Die organische Phase wird abgetrennt, über Natriumsulfat getrocknet, filtriert und eingedampft. Nach Umkristallisieren aus Methylcyclohexan erhält man 4,8 g (58 %) 1-Benzyloxy-4-[3-(1-pyrazolyl)prop-1-enyl]benzol vom Schmelzpunkt 91—93°.

b) 4,51 g (15,5 mMol) 1-Benzyloxy-4-[3-(1-pyrazolyl)prop-1-enyl]benzol werden in einer Mischung aus 25 ml Äthanthiol und 25 ml Bortrifluorid-diäthylätherat aufgelöst. Man rührt die erhaltene Mischung während 0,75 Stunden auf 25°, entfernt das überschüssige Thiol durch Eindampfen und verteilt den Rückstand zwischen Essigester und Wasser. Die organische Phase wird abgetrennt, über Natriumsulfat getrocknet, filtriert und eingedampft. Der Rückstand wird unter Eluieren mit Chloroform an Kieselgel chromatographiert. Nach Eindampfen des Eluates erhält man 3,5 g (86 %) 4-[1-Äthylthio-3-(1-pyrazolyl)propyl]phenol als Öl.

c) 3,5 g 4-[1-Äthylthio-3-(1-pyrazolyl)propyl]phenol werden in 100 ml Methylenchlorid aufgelöst. Man versetzt mit 2,81 g m-Chlorperbenzoesäure und rührt die Lösung während 0,25 Stunden. Nach Waschen der Mischung mit gesättigter Natriumbicarbonatlösung wird die Methylenchloridphase abgetrennt, über Natriumsulfat getrocknet, filtriert und eingedampft. Das erhaltene entsprechende Sulfoxid (3,5 g) wird in 100 ml Toluol aufgelöst. Die erhaltene Lösung wird während 24 Stunden unter Rückfluß zum Sieden erhitzt und anschließend zur Trockne eingedampft. Der Rückstand wird unter Eluieren mit Chloroform/Hexan (9 : 1) an Kieselgel chromatographiert. Nach Eindampfen des Eluates erhält man 0,55 g (22 %) 4-[3-(1-Pyrazolyl)prop-1-enyl]-phenol als gelben kristallinen Festkörper vom Schmelzpunkt 146—152°.

Beispiel 20

In Analogie zu den Angaben in Beispiel 1 erhält man aus 2,6 g 4-[2-(1-Pyrazolyl)äthylthio]phenol 2,45 g (46%) 1-Isopropylamino-3-[4-[2-(1-pyrazolyl)äthylthio]phenoxy]-2-propanol-Hydrogenmaleat vom Schmelzpunkt 84–86°.

Das Ausgangsmaterial kann wie folgt hergestellt werden:

3,15 g (25 mMol) 4-Mercaptophenol in 75 ml Dimethylformamid werden mit 1,2 g (25 mMol) Natriumhydrid (50%ige Öldispersion) behandelt. Die Mischung wird während 5 Minuten gerührt, mit 6,65 g (25 mMol) 1-[2-(p-Toluolsulfonyloxy)äthyl]pyrazol in 20 ml Dimethylformamid versetzt und während 3 Stunden bei Raumtemperatur gerührt. Nach Entfernen des Lösungsmittels wird der Rückstand zwischen Essigester und Wasser verteilt. Die organische Phase wird abgetrennt, über Natriumsulfat getrocknet, filtriert und eingedampft. Nach Umkristallisieren des erhaltenen Festkörpers aus Toluol erhält man 2,6 g (47%) 4-[2-(1-Pyrazolyl)äthylthio]phenol vom Schmelzpunkt 87–91°.

Beispiel 21

In Analogie zu den Angaben im ersten Absatz von Beispiel 1 erhält man aus 1,90 g 4-[2-(1 H-Indazol-1-yl)äthoxy)phenol 1,50 g (49%) 1-[4-[2-(1 H-Indazol-1-yl)äthoxy]phenoxy]-3-isopropylamino-2-propanol-hydrochlorid vom Schmelzpunkt 121–123° (aus Essigester).

Das Ausgangsmaterial kann wie folgt hergestellt werden:

a) 2,95 g (25 mMol) Indazol werden in Analogie zu den Angaben im Beispiel 15 a) mit 7,68 g (25 mMol) 1-Benzyloxy-4-(2-bromäthoxy)benzol [hergestellt wie in Beispiel 1 a) beschrieben] umgesetzt. Man erhält 4,5 g (52%) 1-Benzyloxy-4-[2-(1 H-Indazol-1-yl)äthoxy]benzol vom Schmelzpunkt 81–83° (aus Hexan).
b) Die erhaltene Benzyloxyverbindung wird in Analogie zu den Angaben in zweiten Absatz von Beispiel 8 mit 48%igem Bromwasserstoff in Eisessig debenzyliert. Man erhält 1,9 g (58%) 4-[2-(1 H-Indazol-1-yl)äthoxy]phenol vom Schmelzpunkt 124–125° (aus Toluol).

Beispiel 22

In Analogie zu den Angaben im ersten Absatz von Beispiel 1 erhält man aus 5,1 g 4-[2-(2 H-1,2,3-Triazol-2-yl)äthoxy]phenol 4,50 g (51%) 1-Isopropylamino-3-[4-[2-(2 H-1,2,3-Triazol-2-yl)äthoxy]-phenoxy]-2-propanol-hydrochlorid vom Schmelzpunkt 119–122° (aus Essigester).

Das Ausgangsmaterial kann wie folgt hergestellt werden:

a) 5,0 g (72,5 mMol) 1,2,3-Triazol werden in Analogie zu den Angaben in Beispiel 15 a) mit 22,25 g (72,5 mMol) 1-Benzyloxy-4-(2-bromäthoxy)benzol [hergestellt wie in Bespiel 1 a) beschrieben] umgesetzt. Man erhält 21,6 g eines Festkörpers, der aus einer Mischung von Isomeren besteht. Dieser Festkörper wird mit siedendem Petroläther (60–80°) extrahiert. Man filtriert und läßt das Filtrat abkühlen. Nach beendeter Kristallisation erhält man 9,54 g (45%) 1-Benzyloxy-4-[2-(2 H-1,2,3-Triazol-2-yl)äthoxy]benzol vom Schmelzpunkt 97–100°.
b) Die erhaltene Benzyloxyverbindung wird in Analogie zu den Angaben in Beispiel 1 b) durch katalytische Hydrierung debenzyliert. Man erhält 5,3 g (81%) 4-[2-(2 H-1,2,3-Triazol-2-yl)-äthoxy]phenol vom Schmelzpunkt 63–66° (aus Diäthyläther).

Beispiel 23

675 mg (3 mMol) 1-Isopropylamino-3-(4-hydroxyphenoxy)-2-propanol in 6 ml Dimethylformamid werden mit 288 mg (6 mMol) Natriumhydrid (50%ige Öldispersion) behandelt. Man rührt die Mischung während 5 Minuten, versetzt mit 459 mg (3 mMol) 1-Chlormethylpyrazol-hydrochlorid, rührt während 0,5 Stunden bei 60° und dampft zur Trockne ein. Der Rückstand wird zwischen 2 N-Natronlauge und Methylenchlorid verteilt. Die organische Phase wird abgetrennt, mit Wasser gut gewaschen, über Natriumsulfat getrocknet, filtriert und eingedampft. Der Rückstand wird in Analogie zu den Angaben im ersten Abschnitt von Beispiel 1 mit Maleinsäure behandelt. Man erhält 700 mg (56%) 1-Isopropylamino-3-/4-[(1-pyrazolyl)methoxy]phenoxy/-2-propanol-Hydrogenmaleat vom Schmelzpunkt 84–87° (aus Isopropanol).

Beispiel 24

In Analogie zu den Angaben in Beispiel 18 erhält man aus 3,02 g 1-Chlormethyl-4-chlorpyrazol 5,6 g (61%) 1-[4-[(4-Chlor-1-pyrazolyl)methoxy]phenoxy]-3-isopropylamino-2-propanol-Hydrogenfuma-

rat vom Schmelzpunkt 82–85° (aus Äthanol). Das entsprechende Hydrogenoxalat besitzt einen Schmelzpunkt von 71–75° (Zersetzung, aus Acetonitril).

Das als Ausgangsmaterial verwendete 1-Chlormethyl-4-chlorpyrazol kann wie folgt hergestellt werden:

a) 7,0 g (68,3 mMol) 4-Chlorpyrazol und 10,5 ml 40%ige Formalinlösung in 70 ml Tetrahydrofuran werden während 2 Stunden bei 25° stehengelassen. Man dampft die Lösung zur Trockne ein und verteilt den Rückstand zwischen Wasser und Methylenchlorid. Die wäßrige Phase wird achtmal mit je 100 ml Methylenchlorid ausgeschüttelt. Die vereinigten Auszüge werden über Natriumsulfat getrocknet, filtriert und eingedampft. Nach Umkristallisieren des Rückstandes aus Toluol/Hexan (1 : 1) erhält man 6,8 g (75%) 1-Hydroxymethyl-4-chlorpyrazol vom Schmelzpunkt 68–71°.

b) 2,65 g (19,9 mMol) 1-Hydroxymethyl-4-chlorpyrazol in 20 ml Methylenchlorid werden tropfenweise zu einer Lösung von 3 ml Thionylchlorid in 10 ml Methylenchlorid bei 0° gegeben. Die Mischung wird während 1 Stunde bei 0° gerührt und anschließend während 2 Stunden bei 25° stehengelassen. Man dampft die Lösung zur Trockne ein und nimmt den Rückstand in Diäthyläther auf. Man filtriert um wenig unlösliches Material zu entfernen und dampft erneut ein. Man erhält 2,8 g (93%) 1-Chlormethyl-4-chlorpyrazol als Öl.

## Beispiel 25

In Analogie zu den Angaben in Beispiel 18 erhält man aus 1,42 g 1-Chlormethyl-4-cyanopyrazol 2,4 g (48%) 1-[4-[2-Hydroxy-3-(isopropylamino)propoxy]phenoxymethyl]-4-pyrazolcarbonitril-p-toluolsulfonat vom Schmelzpunkt 97–100° (aus Isopropanol/Äthanol).

Das als Ausgangsmaterial verwendete 1-Chlormethyl-4-cyanopyrazol kann wie folgt hergestellt werden:

a) 8,5 g (91 mMol) 4-Cyanopyrazol und 10 ml 40%ige Formalinlösung in 20 ml Wasser werden während 2 Stunden bei 25° stehengelassen. Die Mischung wird mit Essigester ausgeschüttelt. Der Essigesterauszug wird über Natriumsulfat getrocknet, filtriert und eingedampft. Nach Umkristallisieren des Rückstandes aus Essigester/Hexan erhält man 10 g (89%) 1-Hydroxymethyl-4-cyanopyrazol vom Schmelzpunkt 110–114°.

b) Die erhaltene Hydroxymethylverbindung wird in Analogie zu den Angaben in Beispiel 24 b) mit Thionylchlorid behandelt. Man erhält 11,4 g (98%) 1-Chlormethyl-4-cyanopyrazol als Öl.

## Beispiel 26

In Analogie zu den Angaben im ersten Absatz von Beispiel 1 erhält man aus 333 mg 4-[(2H-1,2,3-Triazol-2-yl)methoxy]phenol 350 mg (59%) 1-Isopropylamino-3-[4-[(2H-1,2,3-Triazol-2-yl)methoxy]phenoxy]-2-propanol-hydrochlorid vom Schmelzpunkt 141–144° (aus Isopropanol).

Das Ausgangsmaterial kann wie folgt hergestellt werden:

a) 4,0 g (58 mMol) 1,2,3-Triazol und 15 ml 40%ige Formalinlösung werden während 2 Stunden bei 25° stehengelassen. Die erhaltene Lösung wird anschließend zehnmal mit je 50 ml Methylenchlorid ausgeschüttelt. Die vereinigten Methylenchloridauszüge werden über Natriumsulfat getrocknet, filtriert und zur Trockne eingedampft. Der halbfeste Rückstand wird in möglichst wenig siedendem Diäthyläther aufgelöst. Man läßt während 2 Tagen bei 0° stehen und erhält 1,55 g (27%) 2-Hydroxymethyl-1,2,3-triazol vom Schmelzpunkt 63–67°.

b) 0,99 g (10 mMol) 2-Hydroxymethyl-1,2,3-triazol werden in 5 ml Thionylchlorid aufgelöst. Nach 5 Minuten dampft man bei einer Temperatur unterhalb 25° unter vermindertem Druck zur Trockne ein. Das erhaltene rohe 2-Chlormethyl-1,2,3-triazol-hydrochlorid wird in 5 ml Dimethylformamid aufgelöst und zu einer Lösung von 2,0 g (10 mMol) 4-Benzyloxyphenol in 20 ml Dimethylformamid, welche vorgängig während 5 Minuten mit 0,96 g (20 mMol) Natriumhydrid (50%ige Öldispersion) gerührt worden ist, gegeben. Man rührt die Mischung während 0,5 Stunden bei 60°, dampft anschließend zur Trockne ein und verteilt den Rückstand zwischen 2 N-Natronlauge und Essigester. Die organische Phase wird abgetrennt, über Natriumsulfat getrocknet, filtriert und eingedampft. Der Rückstand wird in siedendem Petroläther (60–80°) aufgelöst. Man filtriert die noch heiße Lösung und läßt dann langsam abkühlen. Das auskristallisierte Material wird abfiltriert und liefert 1,03 g (37%) 1-Benzyloxy-4-[(1H-1,2,3-triazol-1-yl)methoxy]benzol vom Schmelzpunkt 69–73°. Durch Eindampfen des Filtrates erhält man 613 mg (22%) 1-Benzyloxy-4-[(2H-1,2,3-triazol-2-yl)methoxy]benzol als Öl.

c) Das erhaltene 1-Benzyloxy-4-[(2H-1,2,3-triazol-2-yl)methoxy]benzol wird in Analogie zu den Angaben in Beispiel 1 b) durch katalytische Hydrierung debenzyliert. Man erhält 390 mg (94%) 4-[(2H-1,2,3-Triazol-2-yl)methoxy]phenol.

Beispiel 27

In Analogie zu den Angaben im ersten Absatz von Beispiel 1 erhält man aus 577 mg 4-[(1 H-1,2,3-Triazol-1-yl)methoxy]phenol 350 mg (27%) 1-Isopropylamino-3-[4-[(1 H-1,2,3-triazol-1-yl)methoxy]phenoxy]-2-propanol-Hydrogenmaleat vom Schmelzpunkt 98—101° (aus Isopropanol/Methanol).

Das Ausgangsmaterial kann wie folgt hergestellt werden:

Das nach den Angaben im Beispiel 26 b) hergestellte 1-Benzyloxy-4-[(1 H-1,2,3-triazol-1-yl)methoxy]benzol wird in Analogie zu den Angaben in Beispiel 1 b) durch katalytische Hydrierung debenzyliert. Man erhält 690 mg (99%) 4-[(1 H-1,2,3-Triazol-1-yl)methoxy]phenol vom Schmelzpunkt 157—163°.

Beispiel 28

In Analogie zu den Angaben im ersten Absatz von Beispiel 1 erhält man aus 4-[(1-Pyrazolyl)methylthio]phenol 1-Isopropylamino-3-[4-[(1-pyrazolyl)methylthio]phenoxy]-2-propanol-Hydrogenmaleat vom Schmelzpunkt 83—87° (aus Isopropanol).

Das als Ausgangsmaterial verwendete 4-[(1-Pyrazol-yl)methyl-thio]phenol wird hergestellt, indem man 4-Mercaptophenol mit 1-Chlormethylpyrazol in Dimethylformamid und in Gegenwart von Kaliumcarbonat bei Raumtemperatur umsetzt. Das 4-[(1-Pyrazolyl)methylthio]phenol schmilzt bei 119—122°.

Die folgenden Beispiele betreffen typische pharmazeutische Präparate, enthaltend die erfindungsgemäßen substituierten Phenoxy-aminopropanol-Derivate:

Beispiel A

Tabletten, enthaltend die folgenden Bestandteile, können in herkömmlicher Weise hergestellt werden:

| Bestandteile | mg/Kapsel |
|---|---|
| Substituiertes Phenoxy-aminopropanol-Derivat | 25 |
| Lactose | 106 |
| Stärke | 20 |
| Talk | 9 |
| Kapselfüllgewicht | 160 |

Beispiel B

Kapseln, enthaltend die folgenden Bestandteile, können in herkömmlicher Weise hergestellt werden:

| Bestandteile | mg/Tablette |
|---|---|
| Substituiertes Phenoxy-aminopropanol-Derivat | 25 |
| Lactose | 103 |
| Stärke | 61 |
| Magnesiumstearat | 11 |
| Gesamtgewicht | 200 |

Die erhaltene Kapselabfüllmasse wird zweckmäßigerweise in Hartgelatinekapseln No. 4 abgefüllt.

**Patentansprüche für die Vertragsstaaten: BE, CH, DE, FR, GB, IT, LI, LU, NL, SE**

1. Substituierte Phenoxy-aminopropanol-Derivate der allgemeinen Formel

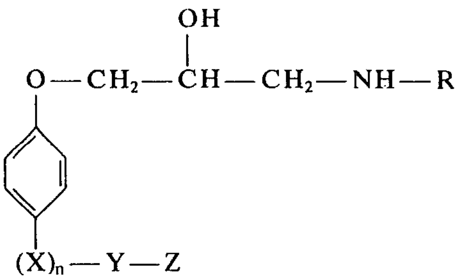

(I)

worin R niederes Alkyl, X ein Sauerstoff- oder Schwefelatom, n die Zahl 0 oder 1, Y Methylen, Äthylen, Propylen oder, wenn n die Zahl 0 bedeutet, auch die Gruppe

$$-CH{=}CH-\overset{*}{C}H_2- \quad (a)$$

wobei die Doppelbindung die trans-Konfiguration aufweist und das mit einem Stern bezeichnete Kohlenstoffatom mit der Gruppe Z verknüpft ist, und Z einen von einem Stickstoffatom aus an die Gruppe Y gebundenen 5-gliedrigen, aromatischen, heterocyclischen Ring, der eines oder mehrere Stickstoffatome als einzige(s) Heteroatom(e) enthält und der durch Halogen, niederes Alkyl, niederes Alkoxy, Phenyl, Cyano oder Carboxamido substituiert sein kann, oder an zwei benachbarten Kohlenstoffatomen mit einem Rest der Formel

$$-(CH_2)_4- \quad (b)$$

oder

$$-CH{=}CH-CH{=}CH- \quad (c)$$

verknüpft sein kann, bedeuten, wobei die als nieder bezeichneten Reste 1—4 Kohlenstoffatome aufweisen,
und pharmazeutisch annehmbare Säureadditionssalze davon.

2. Verbindungen gemäß Anspruch 1, dadurch gekennzeichnet, daß R Isopropyl oder t-Butyl, vorzugsweise Isopropyl, bedeutet, X ein Sauerstoffatom, n die Zahl 1 und Y Methylen bedeuten, und pharmazeutisch annehmbare Säureadditionssalze davon.

3. Verbindungen gemäß Anspruch 1 oder 2, dadurch gekennzeichnet, daß Z 1-Imidazolyl, 1 H-1,2,4-triazol-1-yl, 2 H-1,2,3-Triazol-2-yl, 1-Pyrazolyl oder 4-Halo-1-pyrazolyl, vorzugsweise 2 H-1,2,3-Triazol-2-yl oder 4-Chlor-1-pyrazolyl, bedeutet, und pharmazeutisch annehmbare Säureadditionssalze davon.

4. Verbindungen gemäß einem der Ansprüche 1—3, dadurch gekennzeichnet, daß R Isopropyl, X ein Sauerstoffatom, n die Zahl 1, Y Methylen und Z 2 H-1,2,3-Triazol-2-yl oder 4-Chlor-1-pyrazolyl bedeuten, und pharmazeutisch annehmbare Säureadditionssalze davon.

5. 1-[4-[(4-Chlor-1-pyrazolyl)methoxy]phenoxy]-3-isopropylamino-2-propanol oder pharmazeutisch annehmbare Säureadditionssalze davon.

6. 1-Isopropylamino-3-[4-[(2 H-1,2,3-triazol-2-yl)methoxy]phenoxy]-2-propanol oder pharmazeutisch annehmbare Säureadditionssalze davon.

7. 1-Isopropylamino-3-[4-[2-(1-pyrazolyl)äthoxy]phenoxy]-2-propanol, 1-[4-[2-(1-Imidazolyl)äthoxy]phenoxy]-3-isopropyl-amino-2-propanol, 1-[4-[2-(1-Benzimidazolyl)äthoxy]phenoxy]-3-isopropylamino-2-propanol, 1-Isopropylamino-3-[4-[2-(1 H-1,2,4-triazol-1-yl)äthoxy]phenoxy]-2-propanol, 1-Isopropylamino-3-[4-[2-(4-phenyl-1-pyrazolyl)äthoxy]phenoxy]-2-propanol, 1-Isopropylamino-3-[4-[2-(4-chlor-1-pyrazolyl)äthoxy]phenoxy]-2-propanol, 1-[4-[2-(2 H-Benzotriazol-2-yl)äthoxy]phenoxy]-3-isopropylamino-2-propanol, 1-[4-[2-(4,5,6,7-Tetrahydro-2 H-benzotriazol-2-yl)äthoxy]phenoxy]-3-isopropylamino-2-propanol, 1-[4-[2-(1 H-Indazol-1-yl)äthoxy]phenoxy]-3-isopropylamino-2-propanol oder pharmazeutisch annehmbare Säureadditionssalze davon.

8. 1-Isopropylamino-3-[4-[(1-pyrazolyl)methoxy]phenoxy]-2-propanol, 1-Isopropylamino-3-[4-[(1 H-1,2,3-triazol-1-yl)methoxy]phenoxy]-2-propanol oder pharmazeutisch annehmbare Säureadditionssalze davon.

9. Epoxide der allgemeinen Formel

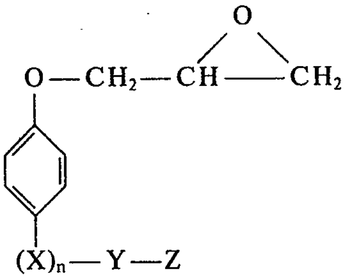

(II)

worin X, Y, Z und n die in Anspruch 1 angegebene Bedeutung besitzen.

10. Phenole der allgemeinen Formel

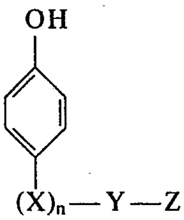

(VI)

worin X, Y, Z und n die in Anspruch 1 angegebene Bedeutung besitzen, mit der Maßgabe, daß Z nicht 1-Imidazolyl bedeutet, wenn n die Zahl 0 und Y Methylen, Äthylen oder Propylen bedeuten, und daß Z nicht 1-Benzimidazolyl bedeutet, wenn n die Zahl 0 und Y Methylen bedeuten.

11. Verbindungen gemäß einem der Ansprüche 1—8 als pharmazeutische Wirkstoffe.

12. Verbindungen gemäß einem der Ansprüche 1—8 als cardio-selektive $\beta$-adrenergische Blocker oder Antihypertensiva.

13. Verfahren zur Herstellung von Verbindungen gemäß einem der Ansprüche 1—8, dadurch gekennzeichnet, daß man

a) ein Epoxid der allgemeinen Formel

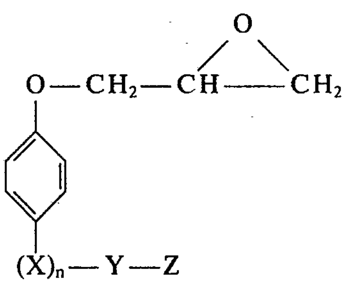

(II)

worin X, Y, Z und n die in Anspruch 1 angegebene Bedeutung besitzen,
mit einem Amin der allgemeinen Formel

$$R-NH_2 \qquad (III)$$

worin R die in Anspruch 1 angegebene Bedeutung besitzt,
umsetzt, oder

b) zur Herstellung einer Verbindung der allgemeinen Formel I, worin X ein Sauerstoffatom, n die Zahl 1 und Y Methylen, Äthylen oder Propylen bedeuten, ein Alkalimetall-Derivat eines Phenols der allgemeinen Formel

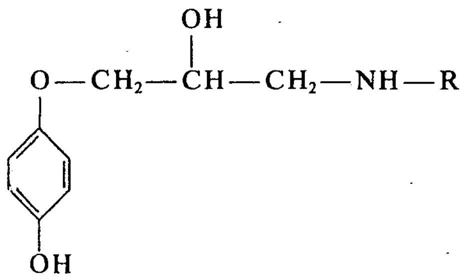

(IV)

worin R die in Anspruch 1 angegebene Bedeutung besitzt, mit einer Verbindung der allgemeinen Formel

$$R^1—Y^1—Z \qquad (V)$$

worin $R^1$ eine Abgangsgruppe und $Y^1$ Methylen, Äthylen oder Propylen bedeuten und Z die in Anspruch 1 angegebene Bedeutung besitzt, umsetzt, und

c) erwünschtenfalls ein erhaltenes Racemat in die optischen Antipoden spaltet, und/oder
d) erwünschtenfalls eine erhaltene Verbindung der allgemeinen Formel I in ein pharmazeutisch annehmbares Säureadditionssalz überführt.

14. Arzneimittel, enthaltend eine Verbindung gemäß einem der Ansprüche 1—8.

15. Cardioselektive $\beta$-adrenergische Blocker oder Antihypertensiva, enthaltend eine Verbindung gemäß einem der Ansprüche 1—8.

**Patentansprüche für den Vertragsstaat: AT**

1. Verfahren zur Herstellung von substituierten Phenoxy-aminopropanol-Derivaten der allgemeinen Formel

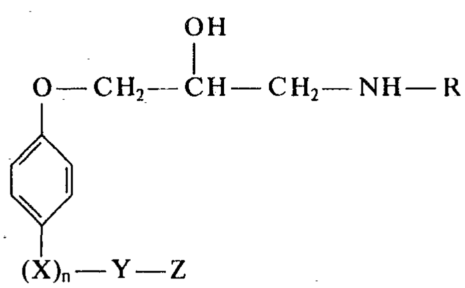

(I)

worin R niederes Alkyl, X ein Sauerstoff- oder Schwefelatom, n die Zahl 0 oder 1, Y Methylen, Äthylen, Propylen oder, wenn n die Zahl 0 bedeutet, auch die Gruppe

$$—CH=CH—\overset{*}{C}H_2— \qquad (a)$$

wobei die Doppelbindung die trans-Konfiguration aufweist und das mit einem Stern bezeichnete Kohlenstoffatom mit der Gruppe Z verknüpft ist, und Z einen von einem Stickstoffatom aus an die Gruppe Y gebundenen 5-gliedrigen, aromatischen, heterocyclischen Ring, der eines oder mehrere Stickstoffatome als einzige(s) Heteroatom(e) enthält und der durch Halogen, niederes Alkyl, niederes Alkoxy, Phenyl, Cyano oder Carboxamido substituiert sein kann, oder an zwei benachbarten Kohlenstoffatomen mit einem Rest der Formel

$$—(CH_2)_4— \qquad (b)$$

oder

$$—CH=CH—CH=CH— \qquad (c)$$

verknüpft sein kann, bedeuten, wobei die als nieder bezeichneten Reste 1—4 Kohlenstoffatome aufweisen, und von pharmazeutisch annehmbaren Säureadditionssalzen davon, dadurch gekennzeichnet, daß man

a) ein Epoxid der allgemeinen Formel

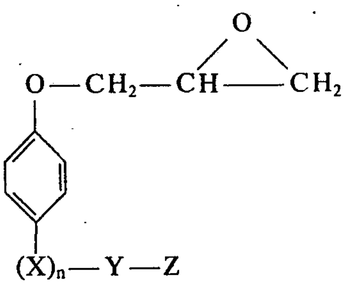

(II)

worin X, Y, Z und n obige Bedeutung besitzen, mit einem Amin der allgemeinen Formel

$R—NH_2$ (III)

worin R obige Bedeutung besitzt, umsetzt, oder

b) zur Herstellung einer Verbindung der allgemeinen Formel I, worin X ein Sauerstoffatom, n die Zahl 1 und Y Methylen, Äthylen oder Propylen bedeuten, ein Alkalimetall-Derivat eines Phenols der allgemeinen Formel

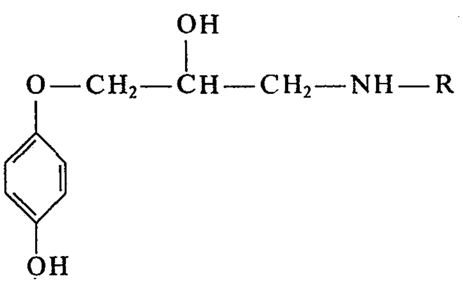

(IV)

worin R obige Bedeutung besitzt, mit einer Verbindung der allgemeinen Formel

$R^1—Y^1—Z$ (V)

worin $R^1$ eine Abgangsgruppe und $Y^1$ Methylen, Äthylen oder Propylen bedeuten und Z obige Bedeutung besitzt, umsetzt, und

c) erwünschtenfalls ein erhaltenes Racemat in die optischen Antipoden spaltet, und/oder

d) erwünschtenfalls eine erhaltene Verbindung der allgemeinen Formel I in ein pharmazeutisch annehmbares Säureadditionssalz überführt.

2. Verfahren gemäß Anspruch 1, dadurch gekennzeichnet, daß man eine Verbindung der in Anspruch 1 definierten allgemeinen Formel I, worin R Isopropyl oder t-Butyl, vorzugsweise Isopropyl, bedeutet, X ein Sauerstoffatom, n die Zahl 1 und Y Methylen bedeuten, herstellt.

3. Verfahren gemäß Anspruch 1 oder 2, dadurch gekennzeichnet, daß man Verbindungen der in Anspruch 1 definierten allgemeinen Formel I, worin Z 1-Imidazolyl, 1 H-1,2,4-Triazol-1-yl, 2 H-1,2,3-Triazol-2-yl, 1-Pyrazolyl oder 4-Halo-1-pyrazolyl, vorzugsweise 2 H-1,2,3-Triazol-2-yl oder 4-Chlor-1-pyrazolyl, bedeutet, herstellt.

4. Verfahren gemäß einem der Ansprüche 1—3, dadurch gekennzeichnet, daß man eine Verbindung der in Anspruch 1 definierten allgemeinen Formel I, worin R Isopropyl, X ein Sauerstoffatom, n die Zahl 1, Y Methylen und Z 2 H-1,2,3-Triazol-2-yl oder 4-Chlor-1-pyrazolyl bedeuten, herstellt.

5. Verfahren gemäß Anspruch 1, dadurch gekennzeichnet, daß man 1-[4-[(4-Chlor-1-pyrazolyl)-methoxy]phenoxy]-3-isopropylamino-2-propanol oder ein pharmazeutisch annehmbares Säureadditionssalz davon herstellt.

6. Verfahren gemäß Anspruch 1, dadurch gekennzeichnet, daß man 1-Isopropylamino-3-[4-[(2 H-1,2,3-triazol-2-yl)methoxy]phenoxy]-2-propanol oder ein pharmazeutisch annehmbares Säureadditionssalz davon herstellt.

**Claims for the Contracting states: BE, CH, DE, FR, GB, IT, LI, LU, NL, SE**

1. Substitued phenoxy-aminopropanol derivatives of the general formula

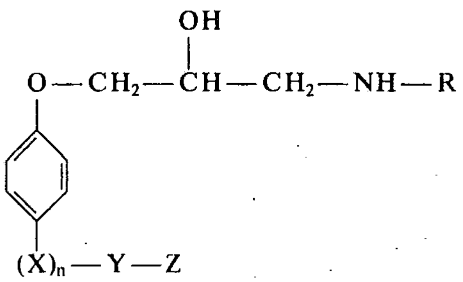

(I)

wherein R represents a lower alkyl group, X represents an oxygen or sulphur atom, n stands for zero or 1, Y represents a methylene, ethylene or propylene group or, when n stands for zero, Y can also represent a group of the formula

$$-CH{=}CH-\overset{*}{C}H_2- \qquad (a)$$

in which the double-bond is trans and the carbon atom marked with an asterisk is linked to Z, and Z represents a 5-membered aromatic heterocyclic ring which contains one or more nitrogen atoms as the sole hetero atom(s), which is linked to Y from a nitrogen atom and which may be substituted by a halogen atom or a lower alkyl, lower alkoxy, phenyl, cyano or carboxamido group or on adjacent carbon atoms by a group of the formula

$$-(CH_2)_4- \qquad (b)$$

or

$$-CH{=}CH-CH{=}CH- \qquad (c)$$

the radicals designated lower containing 1—4 carbon atoms, and pharmaceutically acceptable acid addition salts thereof.

2. Compounds according to claim 1, wherein R represents an isopropyl or tert. butyl group, preferably an isopropyl group, X represents an oxygen atom, n stands for 1 and Y represents a methylene group, and pharmaceutically acceptable acid addition salts thereof.

3. Compounds according to claim 1 or 2, wherein Z represents a 1-imidazolyl, 1 H-1,2,4-triazol-1-yl, 2 H-1,2,3-triazol-2-yl, 1-pyrazolyl or 4-halo-1-pyrazolyl group, preferably a 2 H-1,2,3-triazol-2-yl or 4-chloro-1-pyrazolyl group, and pharmaceutically acceptable acid addition salts thereof.

4. Compounds according to any one of claims 1 to 3, wherein R represents an isopropyl group, X represents an oxygen atom, n stands for 1, Y represents a methylene group and Z represents a 2 H-1,2,3-triazol-2-yl or 4-chloro-1-pyrazolyl group, and pharmaceutically acceptable acid addition salts thereof.

5. 1-[4-[(4-Chloro-1-pyrazolyl)methoxy]phenoxy]-3-isopropylamino-2-propanol or pharmaceutically acceptable acid addition salts thereof.

6. 1-Isopropylamino-3-[4-[(2 H-1,2,3-triazol-2-yl)methoxy]phenoxy]-2-propanol or pharmaceutically acceptable acid addition salts thereof.

7. 1-Isopropylamino-3-[4-[2-(1-pyrazolyl)ethoxy]phenoxy]-2-propanol, 1-[4-[2-(1-imidazolyl)ethoxy]phenoxy]-3-isopropylamino-2-propanol, 1-[4-[2-(1-benzimidazolyl)ethoxy]phenoxy]-3-isopropylamino-2-propanol, 1-isopropylamino-3-[4-[2-(1 H-1,2,4-triazol-1-yl)ethoxy]phenoxy]-2-propanol, 1-isopropylamino-3-[4-[2-(4-phenyl-1-pyrazolyl)ethoxy]phenoxy]-2-propanol, 1-isopropylamino-3-[4-[2-(4-chloro-1-pyrazolyl)ethoxy]phenoxy]-2-propanol, 1-[4-[2-(2 H-benzotriazol-2-yl)ethoxy]phenoxy]-3-isopropylamino-2-propanol, 1-[4-[2-(4,5,6,7-tetrahydro-2 H-benzotriazol-2-yl)ethoxy]phenoxy]-3-isopropylamino-2-propanol, 1-[4-[2-(1 H-indazol-1-yl)ethoxy]phenoxy-3-isopropylamino-2-propanol or pharmaceutically acceptable acid addition salts thereof.

8. 1-isopropylamino-3-[4-[(1-pyrazolyl)methoxy]phenoxy]-2-propanol, 1-isopropylamino-3-[4-[(1 H-1,2,3-triazol-1-yl)methoxy]phenoxy]-2-propanol or pharmaceutically acceptable acid addition salts thereof.

9. Epoxides of the general formula

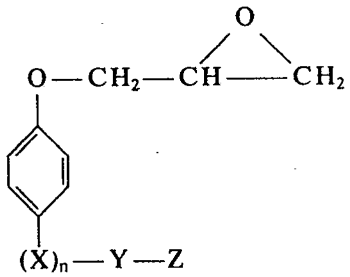

(II)

wherein X, Y, Z and n have the significance given in claim 1.

10. Phenols of the general formula

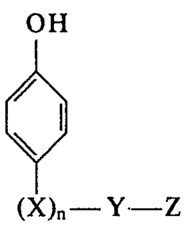

(VI)

wherein X, Y, Z and n have the significance given in claim 1, with the provisos that Z does not represent 1-imidazolyl when n stands for zero and Y represents a methylene, ethylene or propylene group and that Z does not represent 1-benzimidazolyl when n stands for zero and Y represents a methylene group.

11. Compounds according to any one of claims 1 to 8 as pharmaceutically active substances.

12. Compounds according to any one of claims 1 to 8 as cardioselective $\beta$-adrenergic blocking or antihypertensive agents.

13. A process for the manufacture of compounds according to any one of claims 1 to 8, which process comprises

(a) reacting an epoxide of the general formula

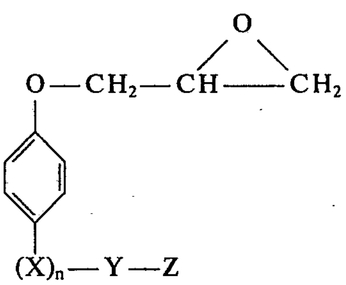

(II)

wherein X, Y, Z and n have the significance given in claim 1,
with an amine of the general formula

$R-NH_2$ (III)

wherein R has the significance given in claim 1,
or

(b) for the manufacture of a compound of formula I in which X represents an oxygen atom, n stands for 1 and Y represents a methylene, ethylene or propylene group, reacting an alkali metal derivative of a phenol of the general formula

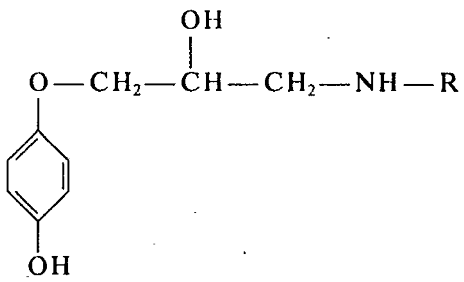

(IV)

wherein R has the significance given in claim 1,
with a compound of the general formula

$$R^1—Y^1—Z \qquad (V)$$

wherein $R^1$ represents a leaving group, $Y^1$ represents a methylene, ethylene or propylene group and Z has the significance given in claim 1,
and/or

(c) if desired, resolving a racemate obtained into the optical antipodes, and/or
(d) if desired, converting a compound obtained of the general formula I into a pharmaceutically acceptable acid addition salt.

14. A medicament containing a compound according to any one of claims 1 to 8.

15. A cardioselective β-adrenergic blocking or antihypertensive agent containing a compound according to any one of claims 1 to 8.

**Claims for the Contracting state: AT**

1. Process for preparing substituted phenoxy-aminopropanol derivatives of the general formula

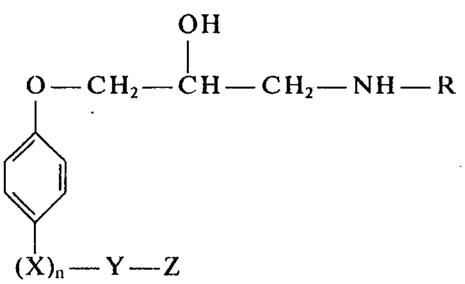

(I)

wherein R represents a lower alkyl group, X represents an oxygen or sulphur atom, n stands for zero or 1, Y represents a methylene, ethylene or propylene group or, when n stands for zero, Y can also represent a group of the formula

$$—CH{=}CH—\overset{*}{C}H_2— \qquad (a)$$

in which the double-bond is trans and the carbon atom marked with an asterisk is linked to Z, and Z represents a 5-membered aromatic heterocyclic ring which contains one or more nitrogen atoms as the sole hetero atom(s), which is linked to Y from a nitrogen atom and which may be substituted by a halogen atom or a lower alkyl, lower alkoxy, phenyl cyano or carboxamido group or on adjacent carbon atoms by a group of the formula

$$—(CH_2)_4— \qquad (b)$$

or

$$—CH{=}CH—CH{=}CH— \qquad (c)$$

the radicals designated lower containing 1—4 carbon atoms, and pharmaceutically acceptable acid addition salts thereof, which process comprises

(a) reacting an epoxide of the general formula

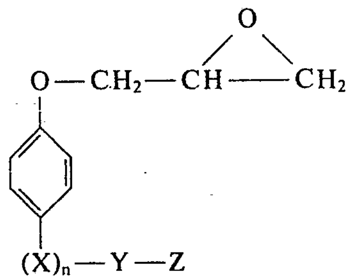

(II)

wherein X, Y, Z and n have the significance given above, with an amine of the general formula

$R-NH_2$ (III)

wherein R has the significance given above, or

(b) for the manufacture of a compound of formula I in which X represents an oxygen atom, n stands for 1 and Y represents a methylene, ethylene or propylene group, reacting an alkali metal derivative of a phenol of the general formula

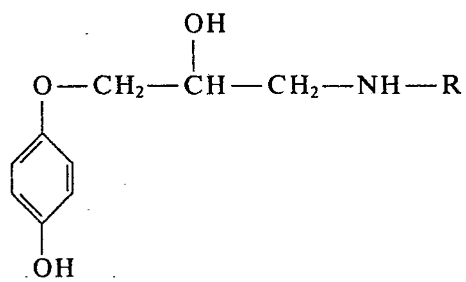

(IV)

wherein R has the significance given above, with a compound of the general formula

$R^1-Y^1-Z$ (V)

wherein $R^1$ represents a leaving group, $Y^1$ represents a methylene, ethylene or propylene group and Z has the significance given above, and/or

(c) if desired, resolving a racemate obtained into the optical antipodes, and/or

(d) if desired, converting a compound obtained of the general formula I into a pharmaceutically acceptable acid addition salt.

2. A process according to claim 1, characterized in that a compound of the general formula I as defined in claim 1 is prepared wherein R represents an isopropyl or tert. butyl group, preferably an isopropyl group, X represents an oxygen atom, n stands for 1 and Y represents a methylene group.

3. A process according to claim 1 or 2, characterized in that a compound of the general formula I as defined in claim 1 is prepared wherein Z represents a 1-imidazolyl, 1H-1,2,4-triazol-1-yl, 2H-1,2,3-triazol-2-yl, 1-pyrazolyl or 4-halo-1-pyrazolyl group, preferably a 2H-1,2,3-triazol-2-yl or 4-chloro-1-pyrazolyl group.

4. A process according to any one of claims 1 to 3, characterized in that a compound of the general formula I as defined in claim 1 is prepared wherein R represents an isopropyl group, X represents an oxygen atom, n stands for 1, Y represents a methylene group and Z represents a 2H-1,2,3-triazol-2-yl or 4-chloro-1-pyrazolyl group.

5. A process according to claim 1 characterized in that 1-[4-[(4-chloro-1-pyrazolyl)methoxy]phenoxy]-3-isopropylamino-2-propanol or a pharmaceutically acceptable acid addition salt thereof is prepared.

6. A process according to claim 1 characterized in that 1-isopropylamino-3-[4-[(2H-1,2,3-triazol-2-yl)methoxy]phenoxy]-2-propanol or a pharmaceutically acceptable acid addition salt thereof is prepared.

**Revendications pour les Etats contractants: BE, CH, DE, FR, GB, IT, LI, LU, NL, SE**

1. Dérivés substitués du phénoxy-aminopropanol de formule générale

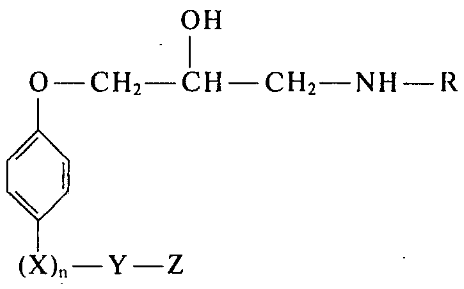

(I)

dans laquelle R représente un groupe alkyle inférieur, X représente un atome d'oxygène ou de soufre, n est égal à 0 ou 1, Y représente un groupe méthylène, éthylène ou propylène ou bien encore, lorsque n est égal à 0, le groupe

$$-CH=CH-\overset{*}{C}H_2- \qquad (a)$$

dans lequel la double liaison est en configuration trans et l'atome de carbone marqué d'un astérisque est relié au groupe Z, et Z représente un noyau hétérocyclique aromatique à 5 chaînons relié au groupe Y par un atome d'azote, qui contient un ou plusieurs atomes d'azote en tant qu'uniques hétéroatomes et qui peut être substitué par un atome d'halogène ou un groupe alkyle inférieur, alcoxy inférieur, phényle, cyano ou carboxamido, ou peut être relié par deux atomes de carbone voisins avec un reste de formule

$$-(CH_2)_4- \qquad (b)$$

ou

$$-CH=CH-CH=CH- \qquad (c)$$

les restes qualifiés d'inférieurs contenant de 1 à 4 atomes de carbone, et leurs sels formés par addition avec des acides acceptables pour l'usage pharmaceutique.

2. Composés selon la rev. 1, caractérisés en ce que R représente un groupe isopropyle ou tert.-butyle, de préférence isopropyle, X représente un atome d'oxygène, n est égal à 1 et Y représente le groupe méthylène, et leurs sels formés par addition avec des acides acceptables pour l'usage pharmaceutique.

3. Composés selon la rev. 1 ou 2, caractérisés en ce que Z représente un groupe 1-imidazolyle, 1 H-1,2,4-triazole-1-yle, 2 H-1,2,3-triazole-2-yle, 1-pyrazolyle ou 4-halogéno-1-pyrazolyle, de préférence 2 H-1,2,3-triazole-2-yle ou 4-chloro-1-pyrazolyle, et leurs sels formés par addition avec des acides acceptables pour l'usage pharmaceutique.

4. Composés selon l'une des rev. 1 à 3, caractérisés en ce que R représente un groupe isopropyle, X un atome d'oxygène, n est égal à 1, Y représente le groupe méthylène et Z un groupe 2 H-1,2,3-triazole-2-yle ou 4-chloro-1-pyrazolyle, et leurs sels formés par addition avec des acides acceptables pour l'usage pharmaceutique.

5. Le 1-[4-[(4-chloro-1-pyrazolyl)-méthoxy]-phénoxy]-3-isopropylamino-2-propanol ou ses sels formés par addition avec des acides acceptables pour l'usage pharmaceutique.

6. Le 1-isopropylamino-3-[4-[(2 H-1,2,3-triazole-2-yl)-méthoxy]-phénoxy]-2-propanol ou ses sels formés par addition avec des acides acceptables pour l'usage pharmaceutique.

7. Le 1-isopropylamino-3-[4-[2-(1-pyrazolyl)-éthoxy]-phénoxy]-2-propanol, le 1-[4-[2-(1-imidazolyl)-éthoxy]-phénoxy]-3-isopropyl-amino-2-propanol, le 1-[4-[2-(1-benzimidazolyl)-éthoxy]-phénoxy]-3-isopropylamino-2-propanol, le 1-isopropylamino-3-[4-[2-(1 H-1,2,4-triazole-1-yl)-éthoxy]-phénoxy]-2-propanol, le 1-isopropylamino-3-[4-[2-(4-phényl-1-pyrazolyl)-éthoxy]-phénoxy]-2-propanol, le 1-isopropylamino-3-[4-[2-(4-chloro-1-pyrazolyl)-éthoxy]-phénoxy]-2-propanol, le 1-[4-[2-(2 H-benzotriazole-2-yl)-éthoxy]-phénoxy]-3-isopropylamino-2-propanol, le 1-[4-[2-(4,5,6,7-tétrahydro-2 H-benzotriazole-2-yl)-éthoxy]-phénoxy]-3-isopropylamino-2-propanol, le 1-[4-[2-(1 H-indazole-1-yl)- éthoxy]-phénoxy]-3-isopropylamino-2-propanol ou leurs sels formés par addition avec des acides acceptables pour l'usage pharmaceutique.

8. Le 1-isopropylamino-3-[4-[(1-pyrazolyl)-méthoxy]-phénoxy]-2-propanol, le 1-isopropylamino-3-[4-[(1 H-1,2,3-triazole-1-yl)-méthoxy]-phénoxy]-2-propanol ou leurs sels formés par addition avec des acides acceptables pour l'usage pharmaceutique.

9. Epoxydes de formule générale

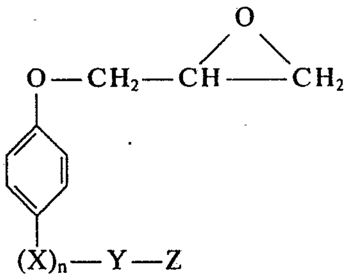

(II)

dans laquelle X, Y, Z et n ont les significations indiquées dans la rev. 1.

10. Phénols de formule générale

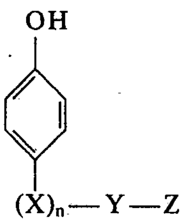

(VI)

dans laquelle X, Y, Z et n ont les significations indiquées dans la rev. 1, étant spécifié que Z ne peut représenter le groupe 1-imidazolyle lorsque n est égal à 0 et Y représente un groupe méthylène, éthylène ou propylène, et que Z ne peut représenter le groupe 1-benzimidazolyle lorsque n est égal à 0 et Y représente un groupe méthylène.

11. Composés selon l'une des rev. 1 à 8, en tant que substances actives pharmaceutiques.

12. Composés selon l'une des rev. 1 à 8, en tant que bloquants bêta-adrénergiques cardiosélectifs ou antihypertensifs.

13. Procédé de préparation des composés selon l'une des rev. 1 à 8, caractérisé en ce que:

a) on fait réagir un époxyde de formule générale

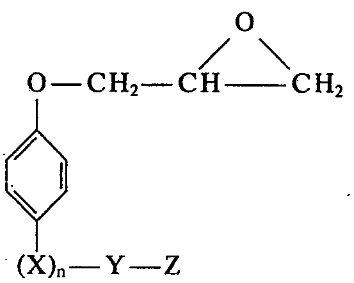

(II)

dans laquelle X, Y, Z et n ont les significations indiquées dans la rev. 1, avec une amine de formule générale

$R—NH_2$ (III)

dans laquelle R a les significations indiquées dans la rev. 1, ou bien

b) pour la préparation d'un composé de formule générale I dans laquelle X représente un atome d'oxygène, n est égal à 1 et Y représente un groupe méthylène, éthylène ou propylène, on fait réagir un dérivé de métal alcalin d'un phénol de formule générale

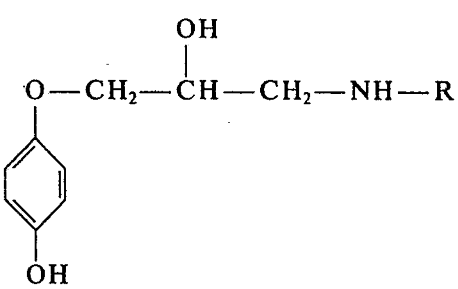

(IV)

dans laquelle R a les significations indiquées dans la rev. 1, avec un composé de formule générale

$$R^1—Y^1—Z \quad (V)$$

dans laquelle $R^1$ représente un groupe éliminable et $Y^1$ un groupe méthylène, éthylène ou propylène, Z ayant les significations indiquées dans la rev. 1, et

c) si on le désire, on résout un racémate obtenu en les antipodes optiques, et/ou

d) si on le désire, on convertit un composé obtenu répondant à la formule générale I en un sel formé par addition avec un acide acceptable pour l'usage pharmaceutique.

14. Médicaments contenant un composé selon l'une des rev. 1 à 8.

15. Bloquants bêta-adrénergiques cardiosélectifs ou anti-hypertensifs contenant un composé selon l'une des rev. 1 à 8.

**Revendications pour l'Etat contractant: AT**

1. Procédé de préparation de dérivés substitués du phénoxy-aminopropanol de formule générale

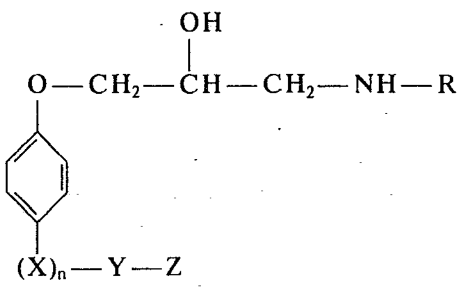

(I)

dans laquelle R représente un groupe alkyle inférieur, X un atome d'oxygène ou de soufre, n est égal à 0 ou 1, Y représente un groupe méthylène, éthylène, propylène ou bien encore, lorsque n est égal à 0, le groupe

$$—CH{=}CH—\overset{*}{C}H_2— \quad (a)$$

dans lequel la double liaison est en configuration trans et l'atome de carbone marqué d'un astérisque est relié au groupe Z, et Z représente un noyau hétérocyclique aromatique à 5 chaînons relié au groupe Y par un atome d'azote, qui contient un ou plusieurs atomes d'azote en tant qu'uniques hétéroatomes et qui peut être substitué par un atome d'halogène ou un groupe alkyle inférieur, alcoxy inférieur, phényle, cyano ou carboxamido, ou peut être relié par deux atomes de carbone voisins avec un reste de formule

$$—(CH_2)_4— \quad (b)$$

ou

$$—CH{=}CH—CH{=}CH— \quad (c)$$

les restes qualifiés d'inférieurs contenant de 1 à 4 atomes de carbone, et de leurs sels formés par addition avec des acides acceptables pour l'usage pharmaceutique, caractérisé en ce que:

a) on fait réagir un époxyde de formule générale

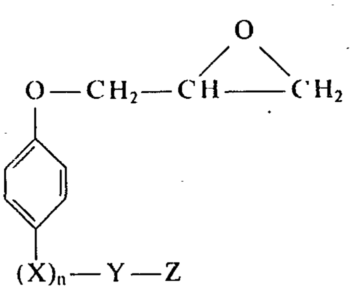

(II)

dans laquelle X, Y, Z et n ont les significations indiquées ci-dessus, avec une amine de formule générale

$R—NH_2$ (III)

dans laquelle R a les significations indiquées ci-dessus, ou bien

b) pour la préparation d'un composé de formule générale I dans laquelle X représente un atome d'oxygène, n est égal à 1 et Y représente un groupe méthylène, éthylène ou propylène, on fait réagir un dérivé de métal alcalin d'un phénol de formule générale

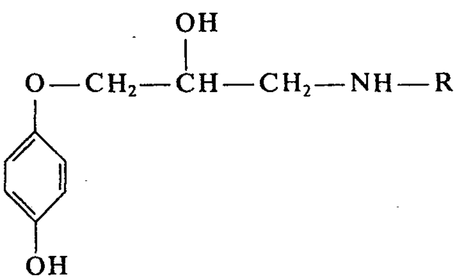

(IV)

dans laquelle R a les significations indiquées ci-dessus, avec un composé de formule générale

$R^1—Y^1—Z$ (V)

dans laquelle $R^1$ représente un groupe éliminable et $Y^1$ un groupe méthylène, éthylène ou propylène, Z ayant les significations indiquées ci-dessus, et

c) si on le désire, on résout un racémate obtenu en les antipodes optiques, et/ou

d) si on le désire, on convertit un composé obtenu, répondant à la formule générale I, en un sel par addition avec un acide acceptable pour l'usage pharmaceutique.

2. Procédé selon la rev. 1, caractérisé en ce que l'on prépare un composé de formule générale I définie dans la rev. 1, dans laquelle R représente un groupe isopropyle ou tert.-butyle, de préférence isopropyle, X représente un atome d'oxygène, n est égal à 1 et Y représente le groupe méthylène.

3. Procédé selon la rev. 1 ou 2, caractérisé en ce que l'on prépare des composés de formule générale I définie dans la rev. 1, dans laquelle Z représente un groupe 1-imidazolyle, 1 H-1,2,4-triazole-1-yle, 2 H-1,2,3-triazole-2-yle, 1-pyrazolyle ou 4-halogéno-1-pyrazolyle, de préférence 2 H-1,2,3-triazolyle-2-yle ou 4-chloro-1-pyrazolyle.

4. Procédé selon l'une des rev. 1 à 3, caractérisé en ce que l'on prépare un composé de formule générale I définie dans la rev. 1, dans laquelle R représente un groupe isopropyle, X un atome d'oxygène, n est égal à 1, Y représente le groupe méthylène et Z un groupe 2 H-1,2,3-triazole-2-yle ou 4-chloro-1-pyrazolyle.

5. Procédé selon la rev. 1, caractérisé en ce que l'on prépare le 1-[4-[(4-chloro-1-pyrazolyl)-méthoxy]-phénoxy]-3-isopropylamino-2-propanol ou un sel de ce composé formé par addition avec un acide acceptable pour l'usage pharmaceutique.

6. Procédé selon la rev. 1, caractérisé en ce que l'on prépare le 1-isopropylamino-3-[4-[(2 H-1,2,3-triazole-2-yl)-méthoxy]-phénoxy]-2-propanol ou un sel de ce composé formé par addition avec un acide acceptable pour l'usage pharmaceutique.